# EUROPEAN PATENT SPECIFICATION

(11) **EP 1 626 732 B1**
(45) Date of publication and mention of the grant of the patent: **03.04.2013**
(21) Application number: 04734525.1
(22) Date of filing: 24.05.2004
(51) Int. Cl.: A61P 25/16, A61P 25/28, A61K 38/08, C07K 14/47, C07K 5/10, C12N 15/113

(54) **MEANS FOR PREVENTING AND TREATING CELLULAR DEATH AND THEIR BIOLOGICAL APPLICATIONS**
MITTEL ZUR PRÄVENTION UND BEHANDLUNG VON ZELLTOD UND IHRE BIOLOGISCHEN ANWENDUNGEN
MOYENS POUR PREVENIR ET TRAITER LA MORT CELLULAIRE ET APPLICATIONS BIOLOGIQUES ASSOCIEES

(30) Priority: 22.05.2003 FR 0306190; 16.12.2003 US 529697 P; 17.03.2004 US 553569 P
(43) Date of publication of application: 22.02.2006
(73) Proprietor: CHIESI FARMACEUTICI S.p.A., 43100 Parma (IT)
(72) Inventor: CHAUVIER, David, 94450 Limeil-Brevannes (FR); BORGNE, Annie, F-75011 Paris (FR); JACOTOT, Etienne, F-75015 Paris (FR); LANGONNE, Alain, F-29260 Saint Meen (FR); LECOEUR, Hervé, F-75014 Paris (FR); REBOUILLAT, Dominique, F-75015 Paris (FR)
(74) Representative: Minoja, Fabrizio
(86) International application number: PCT/EP2004/006288
(87) International publication number: WO 2004/103389

(56) References cited:
- WO-A-02/24720
- WO-A2-02/34201
- MEGURO, TOSHINARI ET AL: "Caspase inhibitors attenuate oxyhemoglobin-induced apoptosis in endothelial cells" STROKE , 32(2), 561-566 CODEN: SJCCA7; ISSN: 0039-2499, 2001, XP008071842
- AOKI, KAZUYA ET AL: "Therapeutic effect of caspase inhibitors in the prevention of apoptosis and reversal of chronic cerebral vasospasm" JOURNAL OF CLINICAL NEUROSCIENCE, vol. 9, no. 6, 2002, pages 672-677, XP008071836 ISSN: 0967-5868
- STEFANIS L ET AL: "Caspase-2 (Nedd-2) processing and death of trophic factor-deprived PC12 cells and sympathetic neurons occur independently of caspase-3 (CPP32)-like activity" JOURNAL OF NEUROSCIENCE 15 NOV 1998 UNITED STATES, vol. 18, no. 22, 15 November 1998 (1998-11-15), pages 9204-9215, XP008047225 ISSN: 0270-6474
- LASSUS PATRICE ET AL: "Requirement for caspase-2 in stress-induced apoptosis before mitochondrial permeabilization." SCIENCE. UNITED STATES 23 AUG 2002, vol. 297, no. 5585, 23 August 2002 (2002-08-23), pages 1352-1354, XP001187581 ISSN: 1095-9203 cited in the application
- DROIN N ET AL: "Involvement of caspase-2 long isoform in Fas-mediated cell death of human leukemic cells" BLOOD 15 MAR 2001 UNITED STATES, vol. 97, no. 6, 15 March 2001 (2001-03-15), pages 1835-1844, XP008047224 ISSN: 0006-4971
- BERGERON L ET AL: "DEFECT IN REGULATION OF APOPTOSIS IN CASPASE-2-DEFICIENT MICE" GENES AND DEVELOPMENT, COLD SPRING HARBOR LABORATORY PRESS, NEW YORK, US, vol. 12, May 1998 (1998-05), pages 1304-1314, XP000952591 ISSN: 0890-9369
- KUMAR S: "Inhibition of apoptosis by the expression of antisense Nedd2." FEBS LETTERS. NETHERLANDS 10 JUL 1995, vol. 368, no. 1, 10 July 1995 (1995-07-10), pages 69-72, XP001187587 ISSN: 0014-5793
- TROY CAROL M ET AL: "Caspases on the brain." JOURNAL OF NEUROSCIENCE RESEARCH. UNITED STATES 15 JUL 2002, vol. 69, no. 2, 15 July 2002 (2002-07-15), pages 145-150, XP008029695 ISSN: 0360-4012 cited in the application
- CHAUVIER ET AL.: CELL DEATH AND DIFFERENTIATION, vol. 14, 29 September 2006 (2006-09-29), pages 387-391,

## Description

The invention relates to means, methods and products, for blocking preventing or treating cell death, particularly neuronal cell death.

Neuronal cell death occurs during embryogenesis to remove excess of neurons to ensure appropriate pre- and post-synaptic connections and to allow formation of a functional adult brain.

Besides post-mitotic death related to normal ageing, environmental or genetic mutational factors may induce neuronal death in the adult human during acute injuries (for instance, hypoxia-ischemia, stroke, spinal cord injury, trauma) or chronic neurodegenerative diseases.

Cell death associated with these disorders may occur by three distinct mechanisms, exhibiting morphological and biochemical features of necrosis, autophagy or apoptosis. Both physiological and pathological neuronal deaths are often associated with defective apoptosis regulation, and signalling pathways that lead to this active cell suicide mechanism may be divided in cysteinyl aspartate-specific protease (caspase)-dependent versus caspase-independent pathways in mammalian cells.

Neuronal apoptosis is an active cell suicide mechanism that can be divided into sequential phases, including initiation, decision, execution, and degradation. This cascade of events is driven by the activation of a specific machinery, that involve both the activation of cysteine-dependent aspartate-specific proteases (caspases) and the mitochondrion which may act as a decisive (or amplifier) regulatory organelle. Indeed, mitochondrial alterations include loss of mitochondrial inner membrane electrochemical gradient (ΔΨₘ) and release of apoptogenic factors such as cytochrome c, Smac/Diablo and Apoptosis Inducing Factor. Once released from mitochondria, these effectors trigger caspase-dependent and/or caspase-independent cytoplasmic and nuclear dismantling. Hence, mitochondrial factors combined with caspases contribute to the degradation phase of apoptosis, resulting in cell shrinkage, nuclear condensation, emission of apoptotic bodies and appearence of "eat-me" signals such as phosphatidyl-serines translocation to the outer leaflet of the plasma membrane. In the absence of phagocytes, cells engaged in apoptosis finally undergo non-specific plasma membrane disruption termed secondary necrosis.

The respective contribution of mitochondria, caspases and other events during neuronal apoptosis is still not elucidated, particularly with respect to a given death inducer/cellular type couple.

Until recently, apoptosis and necrosis of neuronal cells have been mainly investigated by two types of approaches: the first group of (biochemical-) techniques evaluates late events of neuronal death generally by colorimetric evaluation of mitochondrial succinate dehydrogenase activity (MTT assay) or extracellular release of lactate dehydrogenase activity (LDH assay). These routine monoparametric quantitative techniques do not give information concerning the mechanism of cell death and cannot be combined with the detection of other biochemical processes.

More recently, some neuron-adaptated cell-fractionation protocols were published for the biochemical assessment of cytochrome c translocation by immunoblotting and caspases activation using fluorogenic substrates. Such recent methods give semi-quantitative informations on neuron populations but exclude multiparametric and real-time analysis. The second group of techniques use fluorescence microscopy (FM) read-out to detect organelles's modifications or apoptosis-related proteins. The majority of these FM studies are focused on late nuclear alterations including visualisation of chromatin morphology (Hoechst staining) and/or biochemical detection of DNA fragmentation (TUNEL assay). In few recent FM studies on neurons, immuno-localization of cytochrome c (in fixed cells), were reported, but in contrast to other fields of cell biology, a limited number of studies on neurons used the *in situ* detection of mitochondrial alterations and caspase activation. When applied to cultured primary neurons, FM-based analyses are time-consuming, laborious, and quantification is hampered by cellular body aggregates and overlapping neurite networks. In addition, photo-bleaching of sensitive fluorescent probes could lead to dramatic misleading interpretations and exclude real-time follow-up of early death-related events. Thus, cell biology features of key apoptotic events have not been fully documented and ordered in primary neurons.

WO 02/34201 discloses the use of a retinal perycite apoptosis inhibitor for preventing and/or treating diabetic retinopathy.

The inventors have then developed a complementary and quantitative approach to analyse the dynamics of apoptosis phenomena useful, particularly, for primary cortical neurons, or neuronal cell lines, or non-neuronal cell lines.

Such an approach lead the inventors to develop a new method to organize and analyse the molecular events linked to apoptosis. To evaluate with this method the chronology and hierarchy of apoptosis-related events in neuronal cells, the inventors have elaborated an experimental acute death model to determine the more proximal reversible checkpoint to interfere with apoptotic process and applied said method to this model. Advantageously, this evaluation can be performed on neuronal cells, neuronal cell lines, as well as on non neuronal cells and non neuronal cell lines.

The object of the invention is then to provide a multiparametric analytic and imaging plateform method to identify *in cellula* checkpoint to prevent cell death and to the use thereof for blocking and preventing cellular death.

Another object of the invention is that inventors provides methods to real-time following of one or more apoptotic hallmarks in neurons or cell lines.

Another object of the invention is to provide novel compounds that induce caspase-2 (also called Nedd-2; Ich-1) gene silencing, or inhibit pro-apoptotic caspase-2 activity (or interfere with downstream caspase-2 dependent processes).

Another object of the invention is to provide pharmaceutical compositions and methods of treatments of diseases and injuries where caspase-2 is involved.

The scope of the invention is limited by the appended claims.

Disclosed herein is a method for preventing cell death comprising the determination, depending on a given induction way, in a given cellular type, of the hierarchy of apoptosis-related events and the blocking of the more proximal reversible checkpoint to interfere with apoptotic process.

This method is advantageously carried out by combining rapid quantitative flow cytometry and quantitative/qualitative fluorescence microscopy analyses in neurons. It is also advantageously carried out in non neuronal cells. Said method can also be used on neuronal cell lines.

The use of both technologies enables the co-detection of the decision, effector, early and late degradation phases of apoptosis.

As illustrated by the examples, the invention provides means for developing a reliable real-time flow cytometric monitoring of ΔΨₘ and plasma membranes, nuclear and cell morphological granularity and cell size changes in response to neurotoxic insults including MPTP treatment.

Using specific non-overlapping fluorescent probes, and/or specific antibodies and/or pharmacological agents, the invention provides useful means enabling to study the cell biology of apoptosis and to characterize new protective molecules.

Serum deprivation in neuronal culture was used by the inventors as an experimental model to study neuronal death patways and identify upstream checkpoint. During neuronal development and pathology, neurons that fail to find appropriate targets or metabolites (oxygen, glucose, potassium, neurotrophic or growth factors, nutrients) and sources of target-derived neurotrophic factors undergo apoptotic cell death (Deckwerth et al., 1996; Deshmuck *et al*., 1996 and 1998; Lipton, 1999; Plenisla et al., 2001; Chang et al., 2002).

By using said multiparametic and imaging analytic plateform and by studying the selective role of caspases (pharmacological inhibition; small interfering RNA-genes knock-down) in the context of acute serum deprivation (SD)-induced neuronal cell death, the inventors have found that caspase-2 is an upstream regulator of Bax-dependent MMP. Accordingly, the invention particularly relates to the method wherein the checkpoint is caspase-2. The term "caspase" as used in the description and the claims encompasses the various forms obtained by alternative splicing.

As shown by the inventors, early caspase-2 activation is required for mitochondrial Bax translocation, mitochondrial membrane potential (ΔΨₘ) disruption, cytochrome c release-dependent activation of caspase-9/caspase-3, nuclear alterations, phosphatidylserine exposure and final permeabilization of the plasma membrane (PMP).

Also disclosed is that said checkpoint is a caspase.

Also disclosed is that said checkpoint is unrelated caspase activation.

Also disclosed are molecules capable of preventing or blocking caspase-2 activity (and/or caspase-2/bax interaction), to silence caspase-2 expression, and pharmaceutical, compositions useful for treating diseases and injuries where caspase-2 is involved, particularly for treating (hypoxia-) ischemia injuries.

Also disclosed are caspase-2 inhibitors and a method for inhibiting/silencing caspase-2 in neuronal cell death.

Also disclosed is that, the caspase-2 inhibitors are isolated double stranded RNA molecules capable of specifically targeting caspase-2 mRNA to reduce or suppress caspase-2 expression.

Also disclosed are the reduction or suppression of caspase-2 activity in primary neurons or neuronal cell lines, especially from mouse and human origin.

Also disclosed are the reduction or suppression of caspases-2 activity by said inhibitors in non-neuronal cells, including tumor cells.

The double-stranded RNA molecules used to silence caspase-2 expression are duplexes composed of complementary strands of 15-25 nucleotides, preferably 19-25 nucleotides. Preferably, small interfering the end of the strands are stabilized against degradation.

Advantageous siRNA for caspase-2 silencing comprise duplexes of complementary SEQ ID N°1 and SEQ ID N°2. Other advantageous siRNA comprise duplexes of complementary SEQ ID N°6 and SEQ ID N°7.

Also disclosed are the caspase-2 inhibitors which are shRNA. i.e. any shRNA construct based on the sequences of siRNA as above defined that leads *in cellula* to caspase-2 silencing in cells, particularly in neurons and cell lines.

Also disclosed are shRNA contructs which comprise insertion of both SEQ ID N°1 and SEQ, ID N°2, or both SEQ ID N°6 and SEQ ID N°7, or both SEQ ID N°8 and SEQ ID N°9 or both SEQ ID N°10 and SEQ ID N°11.

Said sIRNA or shRNA are obtained by synthesis or produced in the cell double standed.

As illustrated by the examples, siRNA or shRNA-based gene knock-down fully prevents serum-deprivation-induced cortical neuron death.

The invention also relates to the synthesis of each RNA strand, and the combination of the strands to form a double-stranded molecule capable of specifically targeting mRNA caspase-2 *in cellula*.

The synthetized RNA molecules are introduced in human or animal or human origin, under conditions for inhibitory caspase-2 expression. The introduction step comprises use of suitable carriers or is performed by injection.

Alternatively, vectors containing the genetic information for express said RNA are used. Such vectors and also into the scope of the invention.

The inhibitors of the invention block cellular death of either apoptotic or necrotic, or autophagic type.

The inventors have also developed pharmacological (direct inhibition of caspase-2 activity by specific peptide, preferentially but not exclusively pentapeptides) tools to attenuate *in vitro* cell death mediated by caspase-2.Said tools are disclosed in a provisional US pending application.

Since Bax cleavage and caspase-2 activity occur upstream mitochondria in cortical neurons induced to die by serum deprivation and that inhibition of caspase-2 activity leads to survival through inhibition of Bax cleavage, this step of regulation was used by the inventors in order to develop new molecules able to protect cells against death.

As above-mentioned, the inventors have demonstrated that caspase-2 dependent pathways are required in acute models of *in vitro* neuronal death and in vivo stroke. The inventors have shown also that caspase-2 specific inhibition is more efficient to protect neurons *in vivo* in comparison to broad spectrum caspase inhibition. As shown in the Examples, caspase-2 is an upstream major checkpoint for inhibition of neuronal cell death (especially apoptosis) in *in vivo* pathological situation, including hypoxia-ischemia (H-I) injuries.

The invention thus relates to the *in vitro* inhibition of caspase-2 activity with molecule having SEQ ID N°5.

This is a molecule able to disrupt the interaction between Bax and caspase-2 or to prevent caspase-2 dependent Bax cleavage.

Also disclosed are peptides which are derived from Bax sequence with a length of 3 to 40 amino-acids including the sequence IQD (for example: SEQ ID 12-23). Particularly preferred sequences comprise:
SEQ ID N°12: KTGAFLLQGFIQDRAGRMAGETP
SEQ ID N°13: GAFLLQGFIQDRAGRMAGETP
SEQ ID N°14: FLLQGFIQDRAGRMAGETP
SEQ ID N°15: LQGFIQDRAGRMAGETP
SEQ ID N°16: GFIQDRAGRMAGETP
SEQ ID N°17: FIQDRAGRMAGETP
SEQ ID N°18: IQDRAGRMAGETP
SEQ ID N°19: IQDRAGRMAGE
SEQ ID N°20: IQDRAGRMA
SEQ ID N°21: IQDRAGR
SEQ ID N°22: IQDRA
SEQ ID N°23: IQDR

Also disclosed is any molecule able to disrupt the interaction between Bax and caspase-2 or to prevent caspase-2 dependent Bax cleavage, combined in N-ter ou C-ter with peptidic or non-peptidic molecules producing chimeric molecules capable of entering cells (following or not a specific recognition) in order to disrupt interaction between caspase-2 and Bax.

Also disclosed are molecules combined in N-ter ou C-ter with peptidic or non-peptidic molecules producing chimeric molecules capable of entering cells (following or not a specific recognition) in order to prevent or treat apoptosis, or provide mitochondria-protective cytoprotective effects.

Other peptides molecules derived from molecule able to disrupt the interaction between Bax and caspase-2 or to prevent caspase-2 dependent Bax cleavage have a length of 3 to 10 amino-acids including the sequence IQD combined in N-ter ou C-ter with marker (for example: fluorogenic (AMC, AFC, PE...), colorimetric (pNA...) or bioluminescent substrates, radioisotopes...) .

This is another object of the invention to provide pharmaceutical compositions containing specific caspase-2 inhibitors.

The pharmaceutical compositions of the invention comprise a therapeutically effective amount of at least one caspase-2 inhibitor as above defined, in association with a pharmaceutically acceptable carrier.

The invention particularly relates to pharmaceutical compositions comprising siRNA or shRNA molecules such as above defined.

It also relates to pharmaceutical compositions comprising an effective amount of SEQ ID N°5.

Also disclosed are the pharmaceutical compositions comprising an effective amount of at least one molecule able to disrupt the interaction between Bax and caspase-2 or to prevent caspase-2 dependent. Bax cleavage, particularly to the peptides derived from Bax sequence as above defined, particularly those having sequence SEQ ID N°12 to SEQ ID N°23, and the molecules derived therefrom.

The pharmaceutical compositions according to the invention are are advantageously intended for administration by oral, local (intracerebroventricular, intracerebral implantation of Gelfoam® impregnated with compounds or pharmaceutical compositions, intracerebral implantation of instrumentation for mechanical delivery, for example) or systemic (for example: intraperitoneal, intravenous....) administration to reduce cell death.

Administration of the inhibitors comprising RNA duplexes is advantageously carried out in line with classical methods for introducing a nucleic acid in a target cell.

Intraperitoneal administration of a caspase-2 specific inhibitor strongly reduces infarct size in rat pups subjected to transient hypoxia-ischemia brain injury.

Said pharmaceutical compositions are particularly useful for the treatment of pathological situation including hypoxia-ischemia (H-I) H-I (ischemia with or without hypoxia/hypoglycaemia) injuries and stroke-like situations (cerebral, renal, cardiac failure, for example).

They are also of great interest for the treatment of pathological situation including cerebral hypoxia-ischemia (H-I) (ischemia with or without hypoxia/hypoglycaemia) injuries and stroke-like situations (cerebral, renal, cardiac failure, for example).

The pharmaceutical compositions of the invention are also useful for the treatment of neuronal death particularly in global or focal H-I (ischemia with or without hypoxia/hypoglycaemia) injuries and stroke-like situations (cerebral, renal, cardiac failure, for example).

They are also particularly advantageous for the treatment of neuronal death particularly in adult or neonatal H-I (ischemia with or without hypoxia/hypoglycaemia) injuries and stroke-like situations (cerebral, renal, cardiac failure, for example).

They are also useful for the treatment of neuronal death particularly in adult or neonatal H-I (ischemia with or without hypoxia/hypoglycaemia) injuries and stroke-like situations (cerebral, renal, cardiac failure, for example).

They can also be used for the treatment of neuronal death particularly in transient or permanent H-I (ischemia with or without hypoxia/hypoglycaemia) injuries and stroke-like situations (cerebral, renal, cardiac failure, for example).

Said pharmaceutical compositions are also useful for the treatment of neuronal death particularly H-I (ischemia with or without hypoxia/hypoglycaemia) injuries and stroke-like situations brain injuries with or without reperfusion situation (cerebral, renal, cardiac failure, for example).

They can be used for the treatment of neuronal death particularly in Middle Cerebral Artery Occlusion (MCAO).

The above defined pharmaceutical compositions are great of interest for the treatment of neuronal death particularly when at least one or more of the following pathological events are combined: global or focal, transient or permanent, adult or neonatal H-I (ischemia with or without hypoxia/hypoglycaemia) at cerebral level, or at the level of whole body) with or without reperfusion.

Other applications of the pharmaceutical compositions of the invention comprise their use:
- to prevent and/or treat apoptosis during chronic degenerative diseases e.g. neurodegenerative disease including Alzheimer's disease, Huntingtons' disease, Parkinsons' disease, Multiple sclerosis, amyotrophic lateral sclerosis, spinobulbar atrophy, prion disease, or
- to prevent and/or treat apoptosis during spinal cord injury, or to prevent and/or treat apoptosis resulting from traumatic brain injury, or
- to provide neuroprotective effect, or
- to provide cerebroprotective effect, or
- to prevent and/or treat cytotoxic T cell and natural killer cell-mediated apoptosis associated with autoimmune disease and transplant rejection, or
- to prevent cell death of cardiac cells including heart failure, cardiomyopathy, viral infection or bacterial infection of heart, myocardial ischemia, myocardial infarct, and myocardial ischemia, coronary artery bypass graft, or
- to prevent and/or treat mitochondrial drug toxicity e.g. as a result of chemotherapy or HIV therapy,
- to prevent cell death during viral infection or bacterial infection, or
- to prevent and/or treat inflammation or inflammatory diseases, inflammatory bowel disease, sepsis and septic shock, or
- to prevent cell death from follicule to ovocyte stages, from ovocyte to mature egg stages and sperm (for example, methods of freezing and transplanting ovarian tissue, artificial fecondation), or
- to preserve fertility in women and men after chemotherapy, or
- to preserve fertility in females and males animals, or to prevent and/or treat, macular degenerescence and glaucoma, or to prevent and/or treat acute hepatitis, chronic active hepatitis, hepatitis-B, and hepatitis-C, or
- to prevent hair loss, and said hair loss due-to male-pattern baldness, radiation, chemotherapy or emotional stress, or
- to treat or ameliorate skin damage (due to exposure to high level of radiation, heat, burns, chemicals, sun, and autoimmune diseases), or
- to prevent cell death of bone marrow cells in myelodysplastic symdromes (MDS), or
- to treat pancreatisis, or
- to treat respiratory symdrome, or
- to treat osteoarthitis, rheumatoid arthritis, psoriasis, glomerulonephritis, atheroscerosis, and graft versus host disease, or
- to treat retinal pericyte apoptosis, retinal neurons apoptosis glaucoma, retinal damages resulting from ischemia, diabetic retinopaty, or
- to treat disease states associated with an increase of apoptosis, or
- to prevent cell death in vegetals (for example: plants, flowers, thallophytes (mushrooms, seaweed)...)

According to still another aspect, the invention relates to a method for' blocking or preventing cell death *in vitro* comprising screening therapeutically molecules with respect to cell death, particularly apoptosis.

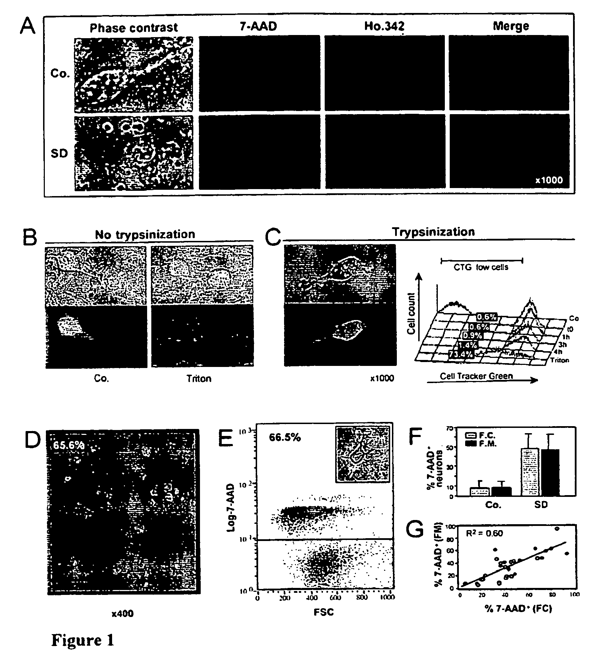

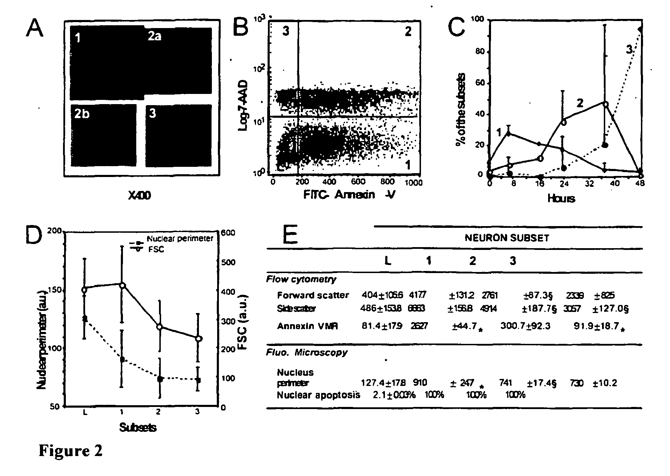

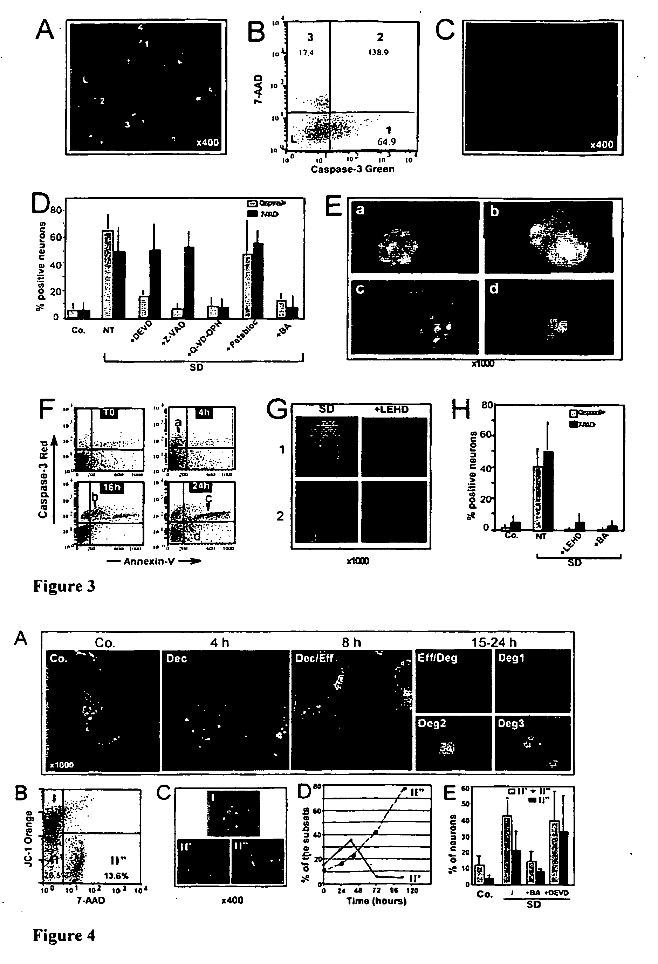

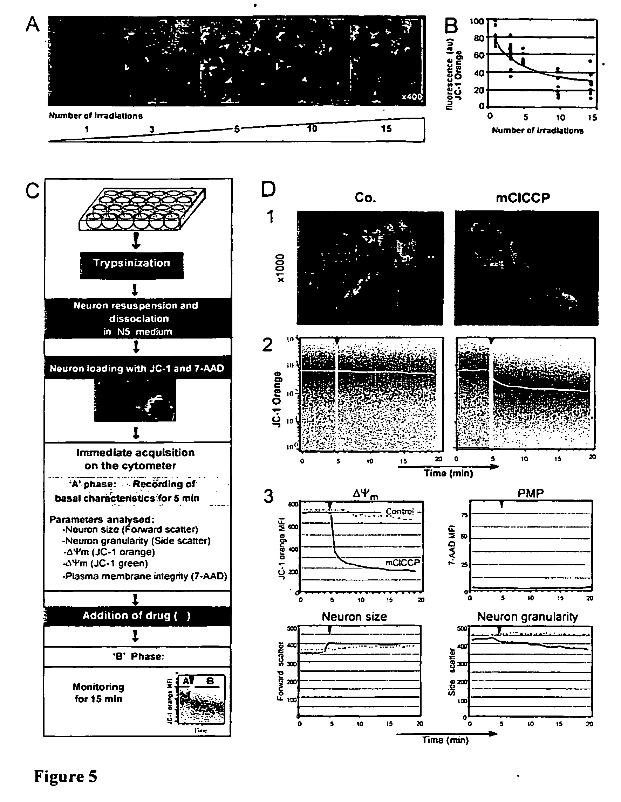

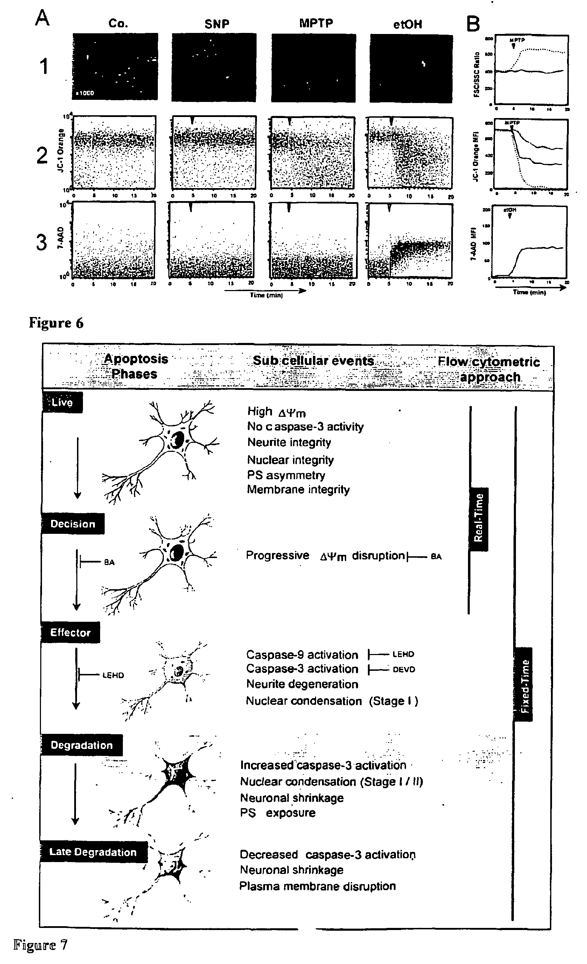

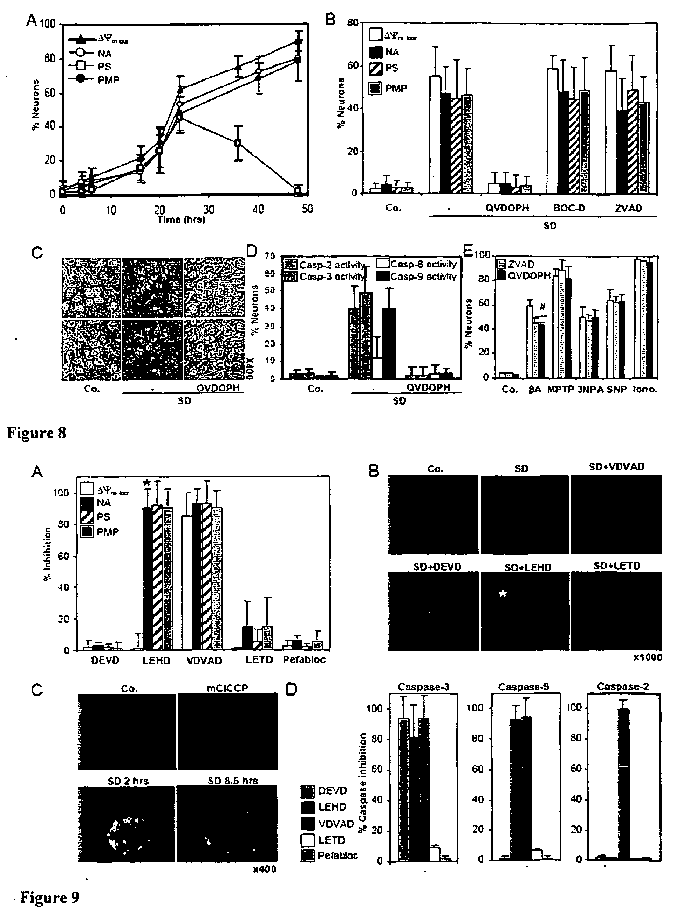

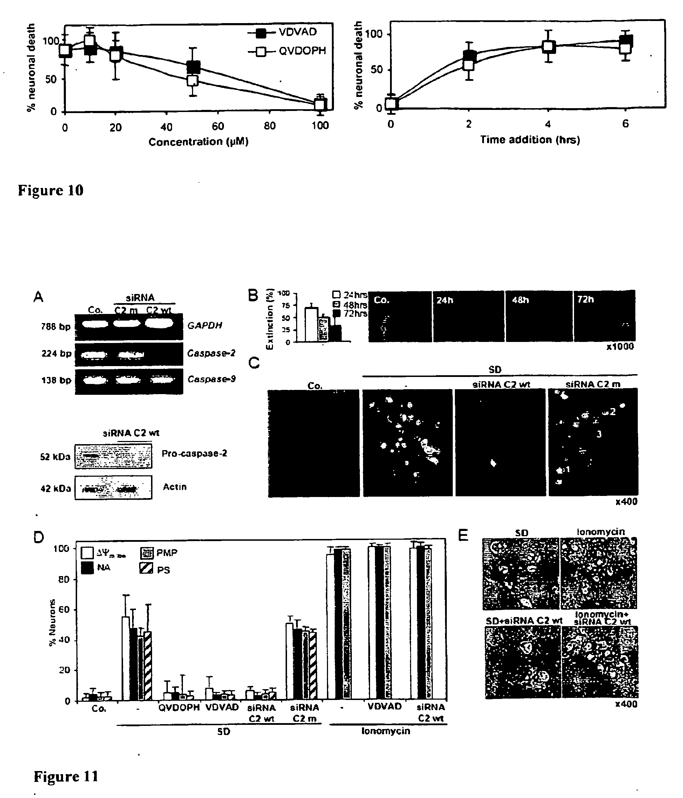

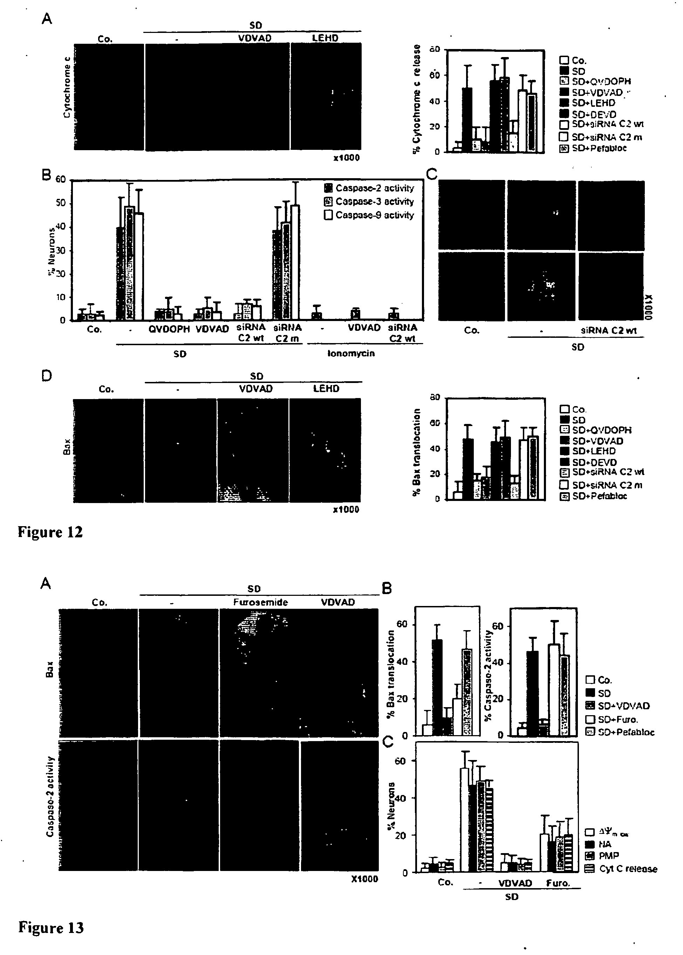

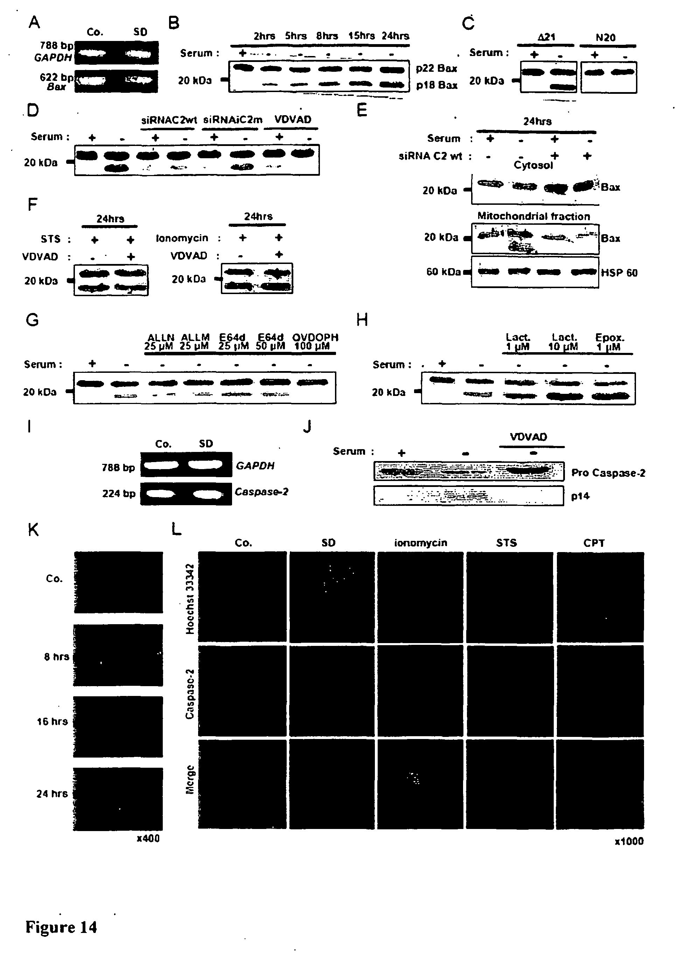

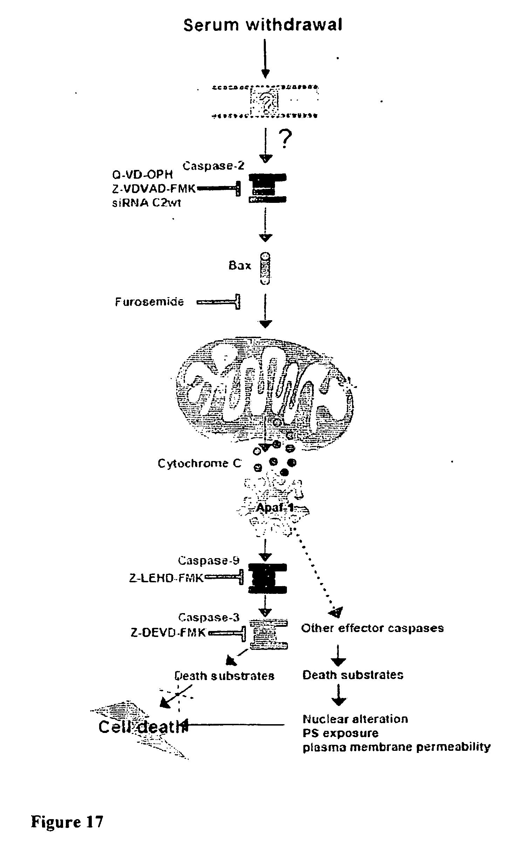

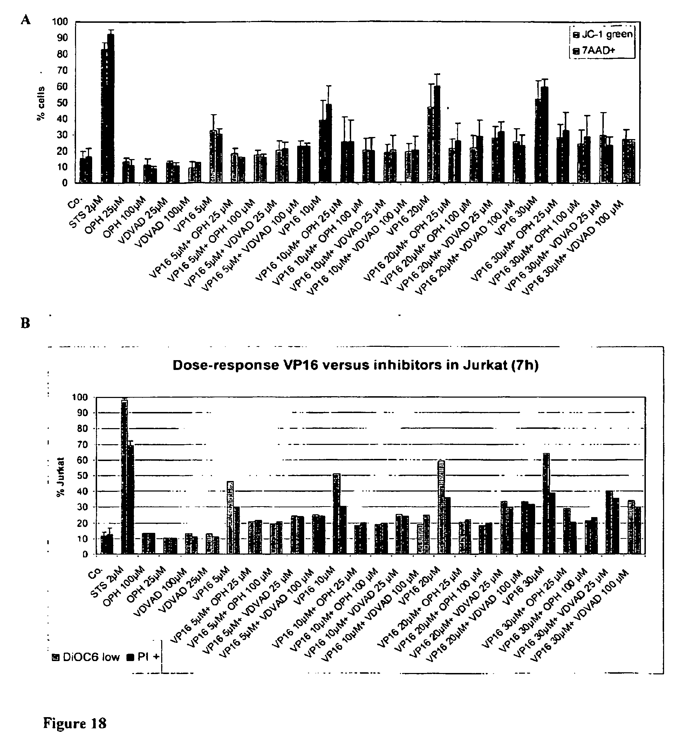

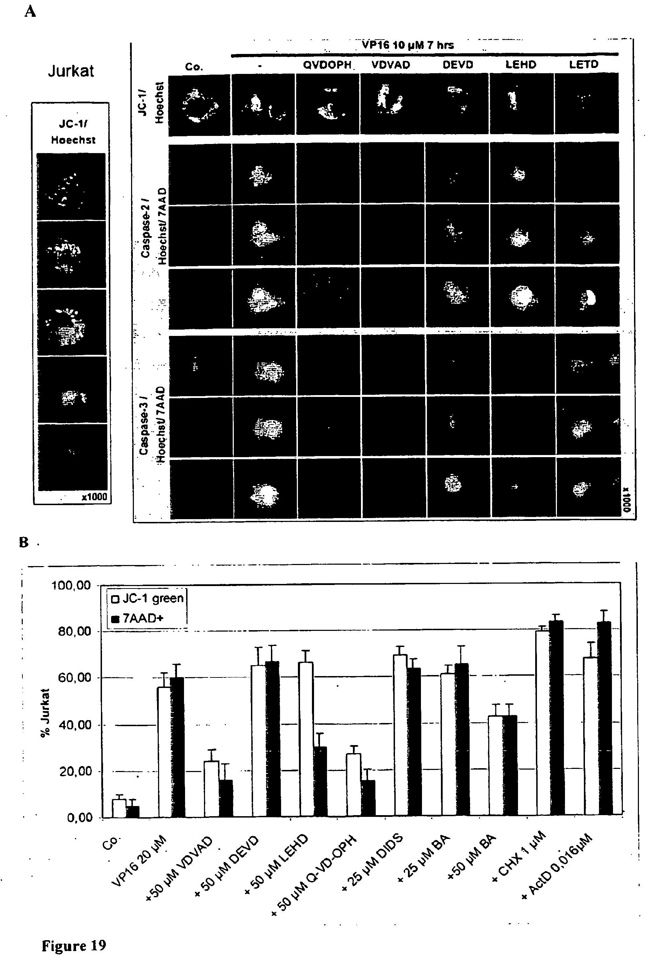

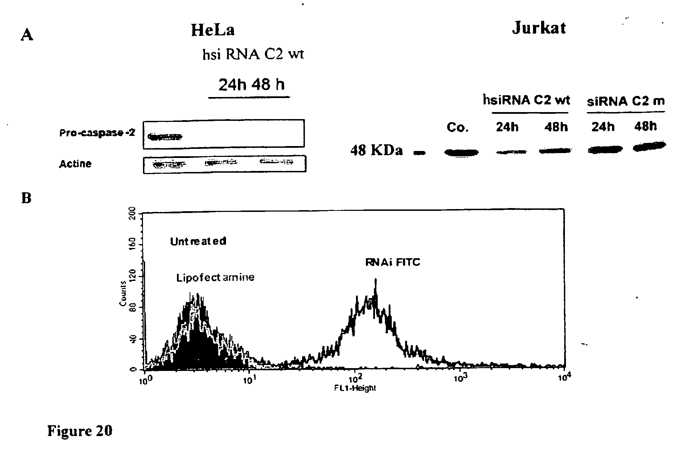

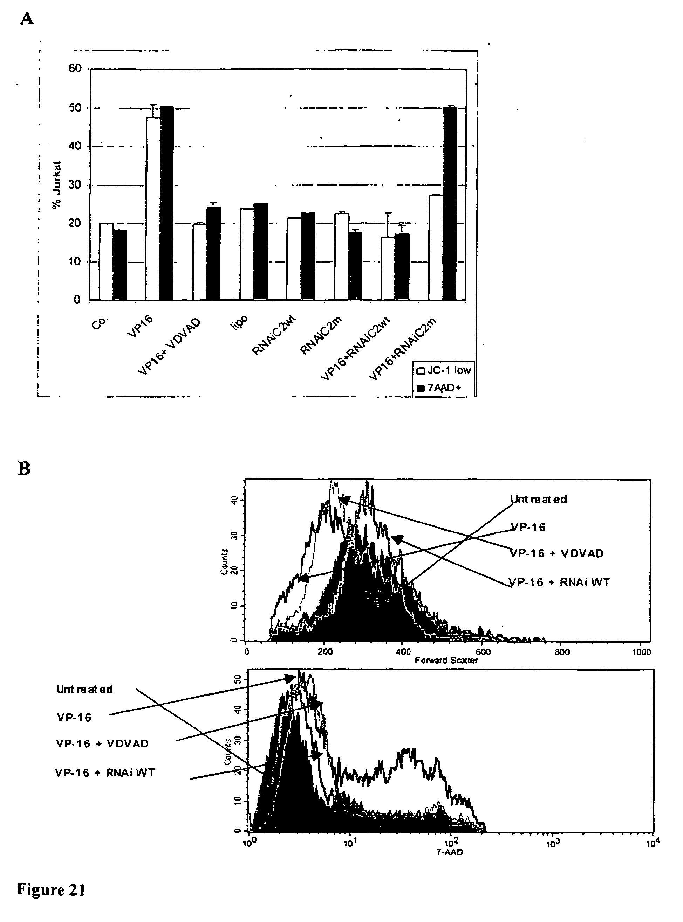

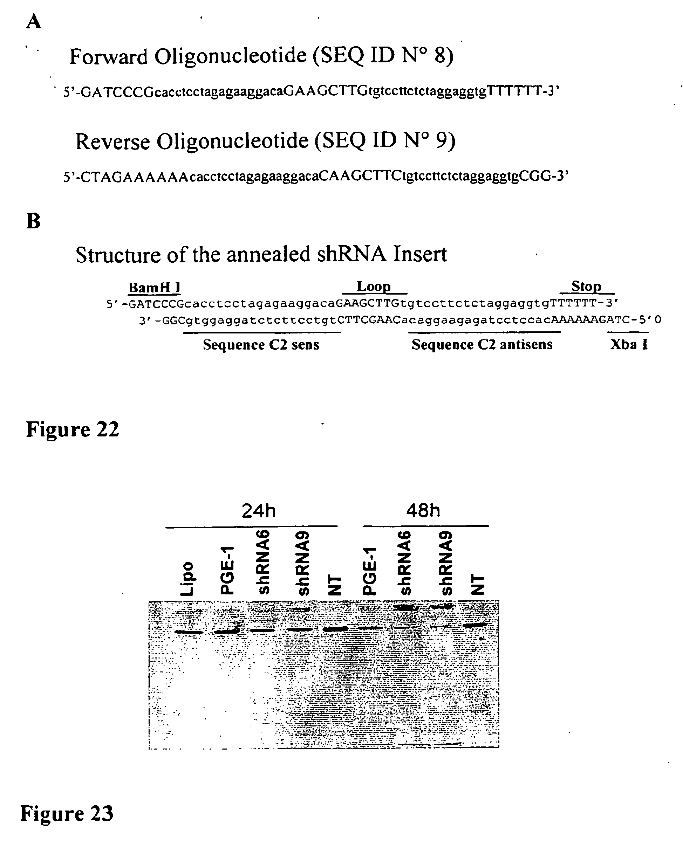

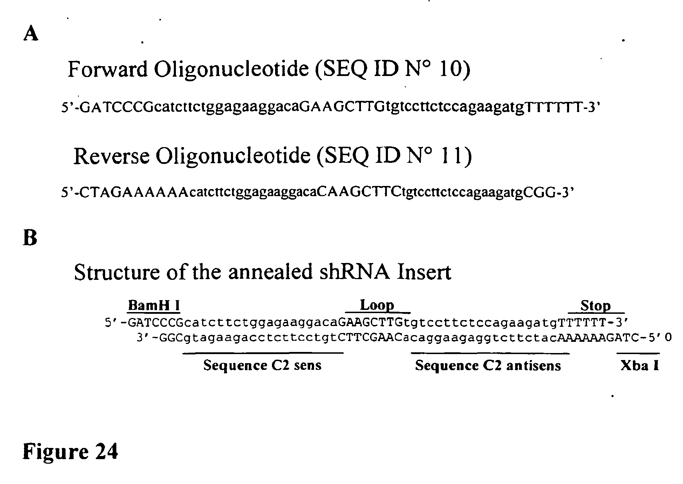

Other characteristics and advantages of the invention are given in the following data with reference to the figures, which represent:
Ref. Figure 1. Combined fluorescence microscopy and flow cytometry detection of plasma membrane permeabilization (PMP) during apoptosis of serum-deprived primary neurons.
   (A) Phase contrast and fluorescence micrographs of cultured cortical neurons submitted or not (Co.) to 24 hours of serum-deprivation (SD). Cells were stained with the cell-permeant fluorescent DNA-ligand Hoechst 33342 (Ho.342, blue fluorescence) and the cell-impermeant fluorescent DNA-intercalent 7-amino-actinomycin D (7-AAD; red fluorescence). Primary cortical neurons representing the dominant phenotype (> 60% of cells submitted to SD) are shown. In SD-neurons, purple fluorescence (blend of red and blue) is indicative of the co-presence of 7-AAD and Hoechst 33342 in condensed nuclei (purple fluorescence in merge), thus correlating PMP to nuclear apoptosis. (B) Effect of triton on PMP and chromatin state of cultured neurons. Cultured neurons were stained with 7-AAD, Hoechst 33342 and the non-toxic CellTracker™ Green fluorescent dye. Representative micrographs of neurons show either merge of phase contrast with both Hoechst 33342 and 7-AAD (upper panels) or CellTracker™ Green alone (lower panels) in the absence (Co.) or after 5 minutes treatment with 0.02% triton. (C) Absence of PMP after trypsinization of neurons. Cultured neurons stained with 7-AAD, Hoechst 33342 and CellTracker™ Green, were submitted to careful detachment trypsin-based protocol (as described in materials and methods). Representative micrographs of trypsinized neurons analysed as in (B) are sown together with FC quantitation of neuron-associated CellTracker™ Green fluorescence at 0, 1, 3 and 4 hours post-trypsinization. (D) FM analysis of PMP and nuclear pyknosis of neurons after SD. Representative micrograph of cultured neuron submitted to 24 hours SD shows merge of phase contrast with both Hoechst 33342 and 7-AAD. Percentage of PMP positive neurons (having purple nuclei) is indicated. (E) FC analysis of PMP. Neurons samples analysed in (D) are subsequently trypsinized and immediately submitted to FC quantification of PMP. Representative dot-plot (FSC /FL3) is shown. FC calculated percentage of PMP positive neurons is indicated. Insert shows a representative phase contrast micrograph of trypsinized cortical neuron. (F) Comparative quantification (n=30) of PMP using FM (optical counting before trypsinization) and FC (automatic counting after trypsinization). (G) Linear correlation between FM- and FC-based PMP quantitation.
**Ref.** **Figure 2****: Combined detection of PMP, PS exposure and nuclear modifications during neuronal apoptosis.**
   (A) Fluorescence micrographs of cultured cortical neurons submitted to 24 hours of SD. Cells were stained with 7-AAD (red fluorescence) and FITC-conjugated annexin V (green fluorescence). Primary cortical neurons are divided in 3 main apoptotic subsets: early apoptotic (annexin V⁺, 7-AAD', subset 1), late apoptotic (annexin V⁺, 7-AAD⁺, subset 2), and end-stage apoptotic (annexin V⁻, 7-AAD⁺, subset 3). (B) FC detection of PMP and PS exposure. Representative dot-plot analysis of neuron subsets 1, 2 and 3. Live neurons exhibit no PS translocation (MFI annexin V = 81.4 +/- 17.9) and are impermeable to 7-AAD (double negative neurons, subset L). (C) FC kinetics of apoptotic subsets appearance throughout SD (n=4; +/- standard deviation). (D) FM-based determination of nuclear perimeter combined to FC-based analysis of neuron size (FSC) among apoptotic subsets. Cultured cortical neurons submitted to 24 hour of SD were stained with Hoechst 33342, 7-AAD and FITC-conjugated annexin V. Multiple fields were acquired during FM observations and samples where then proceeded to FC analysis of cell size using the forward scatter (FSC) parameter. Co-evaluation of nuclear perimeter (n= 15; +/- standard deviation) and FSC (n=7; +/- standard deviation) is presented in per-subset basis. (E). Detailled analysis of FSC, SSC and nuclear features of living (subset L) and dying (subsets 1, 2, 3) neurons. Asterisks denote highly significant (p < 0.0001) and § denote significant (p< 0.05) effects as compared with previous subset.
**Ref.** **Figure 3****: Detection and molecular ordering of activated-caspase-9, caspase-3, PS exposure and PMP**
   (A) Fluorescence micrographs of cultured cortical neurons submitted to 24 hours of SD. Cells were stained with FAM-DEVD-fmk (FLICA; green fluorescence), 7-AAD (red fluorescence) and Hoechst (blue fluorescence). Four distinct phenotypes are detected: Living (Caspase-3⁻, 7-AAD⁻, subset L), early apoptotic (Caspase-3⁺, 7-AAD⁻, subset 1), late apoptotic (Caspase-3⁺, 7-AAD⁺, subset 2), and end-stage apoptotic neurons (Caspase-3⁻, 7-AAD⁺, subset 3). (B) FC co-detection of PMP and caspase-3-like activity. Representative FC dot-plot analysis of neuron subsets L (in blue), 1 (in green), 2 (in yellow) and 3 (in red). (C) Neuroprotection in the presence of the pan-caspase inhibitor Q-VD-OPH. Fluorescence micrographs of cultured cortical neurons are prepared and labeled as in "A". (D) Effects of apoptosis-regulatory compounds on caspase-3 activation and PMP. Neurons were treated with the serine-protease inhibitor Pefabloc, the ANT blocker BA or indicated caspase inhibitors (z-DEVD-fmk, z-VAD-fmk, Q-VD-OPH), and submitted to 24 hour of serum deprivation. Cells were stained with 7-AAD (red fluorescence) and immunostained for activated caspase-3, and then submitted to FC analysis. Results are mean values (± standard deviation) of three independent experiments. (E and F). FM and FC kinetics analysis of caspase-3 activity and PS exposure throughout serum deprivation. Cell were stained with sulforhodamine-conjugated FLICA (red fluorescence), FITC-conjugated annexin V (green fluorescence), and Hoechst (blue fluorescence). Fluorescence micrographs presented in (E) correspond to the subsets "a" to "d" indicated in the dot plots (F). (G) Fluorescence micrographs of cultured cortical neurons submitted to 24 hours of serum withdrawal in the absence (SD) or presence (+LEHD) of the caspase-9 inhibitor z-LEHD-fmk. Cells were stained with Hoechst (blue fluorescence) and co-stained with FAM-LEHD-fmk (FLICA; green fluorescence) in panels 1 or co-stained with FITC-conjugated annexin V (green fluorescence) in panels 2. (H) Hierarchy between ANT-like check-point, caspase-9-like activity and PMP. Neurons were treated with the ANT blocker BA or z-LEHD-fmk and submitted to 24 hour of SD. Cells were stained with 7-AAD (red fluorescence), co-stained with FAM-LEHD-fmk, and then submitted to FM analysis. Results are mean values (± standard deviation) of three independent experiments.
**Ref.** **Figure 4****: Combined detection of ΔΨₘ and PMP in neurons.**
   (A) Fluorescence micrographs of primary neurons cultured for the indicated period, in the absence or presence (24 hr-control; Co) of serum. Cells were stained with Hoechst 33342 (blue fluorescence) and the ΔΨₘ-sensitive dye JC-1 (orange fluorescence of mitochondria with a high ΔΨₘ, green fluorescence of mitochondrial with a low ΔΨₘ). Neurons representing the dominant phenotype are shown (>50%). Dec, decision phase; Eff, effector phase; Deg, degradation phase.
   (B) FC dot-plot analysis of ΔΨₘ and PMP. SD-neurons were stained with 7-AAD and JC-1, trypsinized and immediately submitted to FC analysis. FL2 (JC-1) / FL3 (7-AAD) dot-plots reveal two ΔΨₘ-low neuron subsets: Subset II' impermeant to 7-AAD, and II", 7-AAD positive. (C) FM visualisation of subsets. I, II', II" via the co-detection of ΔΨₘ (JC-1) and plasma membrane permeability (7-AAD). (D) FC time-monitoring of subsets II' and II" in serum-deprived neurons. (E) Neuroprotection by BA but not z-DEVD-fmk evaluated by FC. Histograms indicate either the percentage of ΔΨₘ low neurons (subsets II'+II " , blue histograms), or the percentage of 7-AAD positive neurons (subset II" , black histograms) after 24 hours of SD in the absence or presence of BA or z-DEVD-fmk. Results are the mean of 3 independent experiments (mean +/standard deviation).
**Ref.** **Figure 5****: Real-time detection of ΔΨₘ variation in primary cortical neurons.**
   (A) JC-1 photobleaching induced by FM repetitive irradiations. Fluorescence micrographs of JC-1 stained neurons after 1, 3, 5, 10 and 15 irradiations (1.2 s; 5 Watts). The interval between two irradiations was 1 min. Note the progressive disappearance of the orange fluorescence. (B) Logarithmic regression of JC-1 orange fluorescence intensity assessed on the irradiated field. (C) Protocol for real-time FC monitoring of ΔΨₘ and PMP using JC-1 and 7-AAD probes. Inserted fluorescence micrograph shows a representative visualization of primary neurons co-stained with hoechst, JC-1 and 7-AAD after trypsinization. Note that in these experimental conditions no PMP, no ΔΨₘ loss (neurites and cellular body), nor nuclear condensation are detectable. (D) Application to primary neurons. (D1) Fluorescence micrographs of neurons treated or not (Co.) with mClCCP (100 µM; 30 min). (D2) Real-time FC monitoring of JC-1 orange and JC-1 green fluorescences. The white line corresponds to the mean fluorescence of neurons. (D3) Time-courses of mitochondrial depolarisation (JC-1 orange), PMP (7-AAD) and size (FSC)/granularity (SSC) variations obtained in the same samples (Control, dotted line and mCLCCP-treated, plain line).
**Ref.** **Figure 6****: Real-time FC analysis of ΔΨₘ modifications and PMP induction by different neurotoxic molecules.**
   (A-1) Fixed-time FM of the ΔΨₘ and plasma membrane state. Neurons were treated (or not; Co.) with 0.6 mM SNP or 1 mM MPTP or 20mM ethanol (etOH) for 45 minutes. Cell were stained with JC-1 (orange fluorescence of mitochondria with a high ΔΨₘ, green fluorescence of mitochondria with a high ΔΨₘ), Hoechst (blue fluorescence), and 7-AAD (red fluorescence). (A-2) Real-time FC analysis of ΔΨₘ (JC-1 orange fluorescence) throughout 15 minutes of treatment with medium alone (Co.), 0.6 mM SNP, 1 mM MPTP or 20mM etOH. Orange events correspond to ΔΨₘ high neurons and green events correspond to ΔΨₘ low neurons. (A-3) Real-time FC analysis of PMP (7-AAD fluorescence). (B) Quantitation by real-time FC. (B-1) Analysis of FSC/SSC ratio of MPTP-treated neurons. Red lines correspond to the mean value of FSC/SSC ratio on ΔΨₘ high neurons and dotted green lines correspond to the mean value of FSC/SSC ratio on ΔΨₘ low neurons (as defined in A-2). Plain black line corresponds to the mean value of FSC/SSC ratio on entire neuron population. (B-2) Analysis of JC-1 orange mean fluorescence intensity (MFI) in MPTP-treated neurons. Plain red lines and dotted green lines correspond to JC-1 orange MFI among □□ₘ high and low neurons, respectively. Plain black line corresponds to JC-1 orange MFI on entire neuron population. (B-3) Analysis of the 7-AAD mean fluorescence intensity (MFI) in etOH-treated neurons.
**Ref.** **Figure 7****. Hierarchy of apoptosis-related events during neuronal death induced by SD.**
   The main phases of apoptosis are indicated together with their corresponding subcellular events. An artistic view of neuron behaviour during cell death is presented. Living neurons are drawed with blue nuclei (Hoechst labelling) and red mitochondria (JC-1 labelling; high ΔΨₘ). During the decision phase green mitochondria also appear (JC-1 labelling; low ΔΨₘ) . Effector phase is associated with nuclear shrink and diffuse caspase-3 activation (diffuse pink cytosol). Degradation phase is associated with, neurites brakes, PS exposure (green plasma membrane) and discrete cytosolic activated caspase-3. End stage of degradation is associated with final plasma membrane permeabilization (PMP) leading to nuclear 7-AAD incorporation (red shrinked nuclei). Bax cleavage and translocation appeared upstream of mitochondria but downstream of caspase-2 activity. The point of impact of specific inhibitors is indicated.
**Ref.** **Figure 8****. Pan-caspase inhibition promotes survival of primary cortical neurons induced to die by serum deprivation**
   (A) Time-responses for apoptotic features throughout 48 hr-serum deprived (SD) cortical neurons cultures (DIV6). Kinetics of appearance of neurons with low ΔΨₘ (n=30), nuclear apoptosis (NA) (n=30), permeability of the plasma membrane (PMP) (n=30) or outer leaflet exposure of phosphatidylserine residues (PS) (n=7) are determined by both fluorescence microscopy and cytometry analysis of neurons labeled with J-1, Hoechst 33342, 7-actinomycin D (7-AAD) or FITC conjugated Annexin V, respectively (as previously described in Lecoeur et *al.,* 2004). Note the progressive decrease in PS positive neurons after 24 hrs, since indicating the transition of a ΔΨₘ . low/NA⁺/7-AAD⁺/FITC-annexin V⁺ subset to a terminal ΔΨₘ low/NA⁺/7-AAD⁺/FITC-annexin V⁻ subset (Lecoeur *et al*., 2004).
   (B) Comparative analysis of different pan-caspase inhibitors for neuroprotection. Neurons are subjected to SD concomitantly with the broad spectrum 'caspase inhibitor, Q-VD-OPH, Z-VAD-FMK (ZVAD) or BOC-D-FMK (BOC-D) (all at 100 µM). Histograms indicate the percentage of neurons with low ΔΨₘ (n=12), NA (n=12), PS exposure (n=7) and PMP (n=12) remaining near the control (Co.) level. (C) Q-VD-OPH highly preserves both nuclear morphology and neurites integrity after 24 hr-SD. Representative fields for control (Co.), SD and Q-VD-OPH-treated neurons (100 µM): Upper panels, phase contrast micrographs; lower panels, phase contrast and blue nuclear Hoechst fluorescence are merged. Note the lack of both pronounced neurites disintegration and nuclear condensation/fragmentation in presence of the pan-caspase inhibitor. (D) Four caspases are at least activated during 24 hr-SD. Activation of Caspase-2 (n=14), Caspase-8 (n=3), Caspase-9 (n=8) were detected by using FLICAs, FAM-VDVAD-FMK, FAM-LETD-FMK and FAM-LEHD-FMK, respectively. Caspase-3 activation was detected with either Phycoerythrin-conjugated anti-cleaved caspase-3 polyclonal antibody (n=5) or FAM-DEVD-FMK (n=12), the two approaches being well correlated. Note the low level of caspase-8 activation during SD. All these caspases are completely inactivated by 100 µM Q-VD-OPH. (E) Broad-spectrum caspase inhibitors fail to prevent significantly cortical neurons from NA and PMP induced by β-amyloid (25-35) (βA) , 1-methyl-4-phenyl-1, 2, 3, 6-tetrahydropyridine (MPTP), 3-nitropropionic acid (3-NPA), sodium nitroprusside (SNP) or ionomycin (Iono.). Cortical neurons are treated in absence or presence of 100 µM Z-VAD-FMK (ZVAD) or Q-VD-OPH (QVDOPH) for 24 hrs with ionomycin (6 µM) or βA25-35 (60 µM) ; 48 hrs with MPTP (2 mM), 3-NPA (100 µM) or SNP (500 µM). Neurons displaying both NA and PMP as in (A) are counted. Unpaired Student's t test was performed:# , p = 0.01.
**Ref.** **Figure 9****. Pre mitochondrial caspase-2 like activity is required for cortical neurons apoptotic cell death induced by serum-deprivation**
   (A) Caspase-2 like activity is the most early event detected during SD-induced cell death. Specific inhibitors of caspase-3, caspase-9, caspase-8 and caspase-2 are added at the initiation of SD, respectively at 100 µM: Z-DEVD- FMK (DEVD) (n=8), Z-LEHD-. FMK (LEHD) (n=6), Z-LETD- FMK (LETD) (n=4), Z-VDVAD- FMK (VDVAD) (n=10). Drop in ΔΨₘ, NA, PS exposure and PMP are determined at 24 hrs after JC-1, Hoechst 33342, 7-AAD and FITC conjugated Annexin V stainings, respectively. VDVAD abolishes these hallmarks of apoptosis contrary to DEVD and LETD. While preventing PS exposure, NA and PMP, LEHD does not impair ΔΨₘ drop. Asterisk refers to particular nuclear phenotype in LEHD-treated neurons as depicted in figure 2B. Results are expressed as % of inhibitory effect. (B) Representative fluorescence micrographs for nuclei of neurons treated with specific caspase inhibitors. In contrast to DEVD and LETD, Hoechst 33342-stained nuclei of VDVAD treated-neurons exhibit similar morphology as controls. Nuclei of LEHD-treated neurons have a reduced size corresponding to stage I condensation (according to Susin's classification; Susin et al., 1999). (C) Caspase-2 like activation precedes the ΔΨₘ drop in SD-neurons. Kinetics of caspase-2 activation and ΔΨₘ alterations are evaluated by fluorescence microscopy after co-staining with both FAM-VDVAD-FMK (green) and the ΔΨₘ-sensitive dye CMXRos (red). Caspase-2 like activity (2 hrs) is detected before the progressive ΔΨₘ drop (8.5 hrs). mClCCP (100 µM, 45 min) is used as positive control for complete mitochondrial membrane depolarization. (D) Evaluation of the hierarchy between caspase-2, caspase-3,' caspase-9. Each indicated caspase inhibitor (100 µM) and the serine protease inhibitor Pefabloc (100 µM), is added at the start of SD and caspase-like activities are detected 24 hrs later by using specific FLICAs. Histograms represent % of inhibition for caspase-2, caspase-3, caspase-9 (n=4).
**Ref.** **Figure 10****. Determination of the best pattern for QVDOPH- or VDVAD-induced neuroprotection.**
   Neuronal cell death corresponds to neurons displaying simultaneously low ΔΨₘ (JC-1 green), NA phenotype (Hoechst 33342) and PMP (incorporation of 7-AAD red fluorescence) after 24 hr-SD in presence of caspase inhibitors reported to SD cultures devoid of inhibitors. The left panel shows the dose-response for each inhibitor added at the initiation of SD, and confirms that 100 µM are required for optimal survival. Moreover the protective effects (at t=24 hrs) of either 100 µM VDVAD or QVDOPH progressively decrease when added 2, 4, or 6 hrs after the beginning of SD (right panel). Neurons are counted by fluorescence microscopy (n=3).
**Ref.** **Figure 11****. Genetic proof for caspase-2-mediated apoptosis induced by serum-deprivation: knock-down of caspase-2 by RNA interference approach**
   (A) Gene silencing of murine caspase-2 by small interfering RNA. Neurons at DIV6 are transfected with siRNAs for 6 hrs as described in experimental section prior to further incubation in N5 medium. Upper panels: Endogenous *caspase-2* gene expression at 24 hrs post-transfection is determined by RT-PCR analysis. Note that siRNA C2 wt decreases *caspase-2* expression without any side-effect on other genes (*caspase-9, GAPDH*); lower panels: knock-down of pro-caspase-2 in control neurons by siRNA C2 wt assessed by western blotting. siRNA C2 m is the negative control for gene silencing. *GAPDH* is used as an equal loading control. (B) *In cellula* monitoring of caspase-2 knock-down by immunostaining (10C6). Fluorescence intensity is decreased at 70 % for 24 hrs post-transfection with siRNA C2 wt and progressively recovers at 72 hrs. Fluorescence extinction was followed under FM (5 fields corresponding to 150 randomly chosen cells per condition per experience) by using the Probemeter option of Leica Q Fluoro software. Note that knock-down occurs in all neurons. (C) Caspase-2 activity and cell death parameters are abolished after RNA interference in SD-neurons. Neurons transfected at DIV6 for 6 hrs with siRNAs, are re-cultured in serum-rich medium for 16 hrs, before further 24 hrs conditioning in serum-free medium. Representative fluorescent micrographs: Nuclear condensation/fragmentation (Hoechst; blue), caspase-2 activity (FAM-VDVAD-FMK, green; subset 1) and PMP (7-AAD; red; subset 3); subsets 2 refer to both caspase-2 and 7-AAD positive neurons. Unlike siRNA C2m, siRNA C2 wt prevents caspase-2 activation (n=5). (D) Caspase-2 activity is critical for SD-induced but not ionomycin-induced neuronal cell death. RNA interference prevents other hallmarks of SD-cell death. Quantification of cell death parameters in absence or presence of the indicated inhibitors (100 µM) or siRNAs (n=5). Neurons are treated as in (C) for RNA interference. Drop in ΔΨₘ, NA, PS exposure and PMP are determined by JC-1, Hoechst 33342, 7-AAD and FITC-conjugated Annexin V stainings, respectively. Cell death pathway induced by treatment for 24 hrs with 6 µM of the Ca ²⁺ ionophore is independent of caspase-2 : Note the absence of protection by VDVAD or siRNA C2 wt (n=3). (E) Anaglyphs depicting the protective effects of siRNA C2 wt on PMP (7-AAD incorporation), nuclear (Hoechst 33342; blue) and neurite morphologies after SD in contrast to ionomycin treatment (6 µM, 24 hrs); pink fluorescence results from merge of Hoechst and 7-AAD. Fluorescences are merged with phase contrast images.
**Ref.** **Figure 12****. Caspase-2 is required for both post-mitochondrial cytochrome c release and pre-mitochondrial Bax translocation in 24 hr-serum deprived neurons.**
   (A) VDVAD and siRNA C2 wt reduce post-mitochondrial cytochrome c release. Left panel : Fluorescent micrographs corresponding to the effects of selective caspase inhibitors (100 µM). Neurons treated or not by inhibitors during 24 hr-serum withdrawal are stained with Hoechst 33392 (blue) and the monoclonal antibody (6H2.B4) recognizing the cytochrome c (red). SD triggers cytoplasmic cytochrome c release (diffuse staining) from mitochondria (punctuate staining). Right panel: Corresponding quantitations by FM for cytochrome c release (n=4). For siRNAs assay, neurons at DIV6 are transfected for 6 hrs with siRNAs, then cultured in N5 complete medium prior to further 24 hr-SD. Note that Pefabloc (100 µM), caspase-9 inhibitor LEHD and caspase-3 inhibitor DEVD fail to impair cytochrome c release.
   (B) RNA interference abolishes caspase-2 activation and prevents downstream cytochrome c release-dependent activation of caspases-9 and caspase-3. Neurons are treated as in A, or with 100 µM QVDOPH or VDVAD, and stained with FAM-VDVAD-FMK, FAM-DEVD-FMK and FAM-LEHD-FMK (n=4). Note that cell death pathway induced by ionomycin (6 µM) for 24 hrs is independent of caspase-2 activation in cortical neurons (others caspase activities were not tested). (C) Representative micrographs for *in cellula* caspase-3 inactivation by siRNA C2 wt: Upper panels, blue nuclear Hoechst fluorescence and caspase-3 (cytoplasmic) green fluorescence are merged; Lower panels, red 7-AAD nuclear fluorescence resulting from PMP and cytoplasmic caspase-3 green fluorescence are merged. siRNA C2 wt completely abolished caspase-3 activation, NA and PMP. (D) VDVAD and siRNA C2 wt reduce pre-mitochondrial Bax translocation. Fluorescent micrographs (left panel) and corresponding quantitation (right panel) of the effects of selective caspase inhibitors (100 µM) and siRNAs. Untreated neurons and neurons treated as in A by either inhibitors or siRNAs, are stained with Hoechst 33342 (blue) and the polyclonal Δ21 antibody recognizing Bax (green) at 24 hr-serum withdrawal, prior to be scored under FM (10 fields corresponding to 150-300 randomly chosen cells per condition per experience) (n=4). Bax relocation from cytoplasm (diffuse staining) to mitochondria (punctuate staining) is prevented by VDVAD, QVDOPH and siRNA C2 wt. Note that Pefabloc, LEHD and DEVD fail to impair Bax relocation.
**Ref.** **Figure 13****. Positioning of the protective effects of VDVAD versus furosemide on both Bax translocation and caspase-2 activity**
   (A) Caspase-2 activity is upstream of Bax translocation. Neurons are incubated at the initiation of 24 hr-SD with 2 mM furosemide (Furo.) or 100 µM VDVAD. Neurons are labeled with Hoechst 33342 (Blue) and immunostained for Bax with Δ21 antiboby (upper panel; green) or labeled with FAM-VDVAD-FMK (lower panel; green). Representative fluorescence micrographs show that mitochondrial Bax relocation upon SD is partially prevented by furosemide without impairing caspase-2 activity. In contrast, VDVAD blocks both caspase-2 activation and Bax relocation. (B) Quantitation by FM of neurons displaying Bax relocation or caspase-2 activity (n=4) after treatment as in (A). Pefabloc is negative control. (C) Inhibition of Bax translocation by furosemide results in impairment of ΔΨₘ drop, NA, PMP and cytochrome c release. Neurons treated at the initiation of 24 hr-SD with 2 mM furosemide or 100 µM VDVAD are labeled with JC-1, Hoechst 33342, 7-AAD and monoclonal antibody recognizing the cytochrome c (6H2.B4). Cells are scored by FM (n=3-8).
**Ref.** **Figure 14****. Bax α cleavage is both dependent on cytoplasmic caspase-2 and calpain-independent during SD**
   (A) *Caspase-2* mRNA is analyzed by RT-PCR in 24 hr-SD neurons, revealing no RNA level alteration. *GAPDH* expression is used as loading control. (B) Characterization of Bax cleavage mediated by caspase-2. Neurons are submitted to SD for 2, 5, 8, 15 and 24 hrs and time-course of Bax cleavage is analyzed by Western Blotting using the rabbit polyclonal antibody raised against mouse Bax α deleted for the carboxy terminal 21 amino acids (Δ21). The native p22 Bax is early and progressively cleaved as p18 Bax. (C) Bax cleavage into a 18 kDa form occurs at the N-terminus during SD. Right panel: Comparison of Western Blot analysis of same samples (control and SD-neurons) by using the rabbit polyclonal antibody raised against mouse Bax α deleted for the carboxy terminal 21 amino acids (Δ21) and the rabbit polyclonal antibody raised against a peptide mapping at the amino terminus of Bax α (N20). Both antibodies recognize native Bax while cleaved Bax is only detected with Δ21. (D) The protease inhibitor profile of Bax cleavage is characterized in presence of 100 µM VDVAD or siRNAC2wt (3.8 µg) for 24 hrs SD. VDVAD and siRNA C2 wt prevent Bax cleavage. Bax cleavage depends on both caspase-2 presence and caspase-2 activity. Western Blotting is performed by using the Δ21 antibody. (E) Serum deprivation induces Bax translocation of cleaved p18 Bax into mitochondria, suggesting that p18 Bax is the active form to promote further mitochondrial alterations. Mitochondrial fraction and cytosol of SD-neurons were isolated and translocation of Bax is detected by Western Blotting by using the Δ21 anti-Bax antibody. Mouse anti-HSP60 antibody is used to check mitochondrial fraction. p22 Bax is present in cytosol of 24 hr-SD neurons. However, Bax is partially cleaved at 24 hr-SD in a p18 form which delocalizes from cytosol to mitochondria. siRNA C2 wt or VDVAD prevents integration of p18 Bax into mitochondrial membrane. (F) Bax cleavage mediated by caspase-2 is stimulus-specific in cortical neurons. Neurons are treated for 8, 15 or 24 hrs by staurosporine (STS, 10 µM) or ionomycin in presence or absence of VDVAD (100 µM) prior to immunoblotting analysis using the Δ21 antibody. STS and ionomycin induce caspase-2 independent Bax cleavage in cortical neurons. (G) Bax cleavage is not mediated by calpains. The ability of specific (25 µM ALLN for calpain I; 25 µM ALLM for calpain II) and broad-spectrum (25-50 µM E64d) calpains inhibitors to block 24hr-SD-induced Bax cleavage is examined as in B. These inhibitors are unable to prevent Bax cleavage in contrast to 100 µM QVDOPH: Western Blotting is performed by using the Δ21 antibody. (H) Stabilization of the p18 Bax by inhibition of proteasomal activity. Neurons are cultured in serum-free medium for 24 hrs in absence or presence of proteasome inhibitors: Lactacystin 1- 10 µM (Lact.) and Epoxomycin 10 µM (Epox.). Western Blotting is performed by using the Δ21 antibody. (I-J) Caspase-2 status in 24 hr-SD-neurons: analysis by RT-PCR (I) and Western Blotting (J) using the rat monoclonal anti-mouse caspase-2 antibody (11B4). VDVAD (100 µM) is added at the initiation of SD. Pro-caspase-2 protein content decreases during SD without altering *Caspase-2* mRNA level. *GAPDH* is used as an equal loading control. Pro-caspase-2 protein is not up- or down-regulated but pro-caspase-2 is rather processed as a p14 form in a VDVADase-dependent manner. (K) Atypic caspase-2 localization during SD: Caspase-2 remains diffuse in the cytoplasm of mice primary cortical neurons during SD. Neurons at DIV6 are cultured in serum-free medium for 8, 16 and 24 hrs prior to staining with rat monoclonal anti-mouse caspase-2 antibody (10C6; red). Nuclei are counterstained with 1 µM Hoechst 33342 (blue). (L) Cytoplasmic distribution of caspase-2 during injury is stimulus-dependent. Neurons are treated by cytotoxic concentrations of the Ca²⁺ ionophore ionomycin (6 µM), the kinase inhibitor staurosporine (STS, 10 µM), the topoisomerase I inhibitor camptothecin (CPT, 10 µM) or cultured in serum-free medium for 24 hrs, prior to staining as in (J). Unlike SD, complete nuclear relocation of caspase-2 occurs during treatment with ionomycin and STS. Nuclear relocation is partial for CPT.

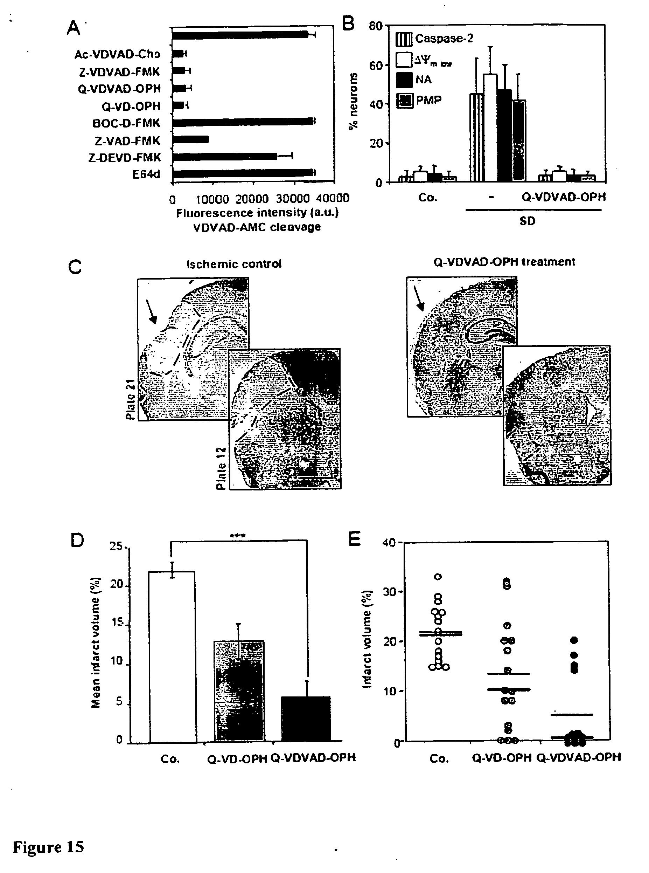

**Figure 15****: Specific caspase-2 inhibition by Qco-VDVAD-dfp provides better neuroprotection than pan-caspase inhibition by Q-VD-OPH against neonatal ischemic brain injury.**
   (A) *In vitro* VDVAD-AMC cleavage by recombinant caspase-2. The cleavage of 50 µM VDVAD-AMC by recombinant human caspase-2 (125 U) was measured after 30 min at 37 °C prior to incubation with selective or pan-caspase inhibitors (2 µM) (n ≥ 2). Caspase-2 cleavage activity is blocked by the prototype compound, Qco-VDVAD-dfp, as efficiently as specific caspase-2 inhibitors (Ac-VDVAD-Cho, Z-VDVAD-FMK) and Q-VD-OPH. While cleavage inhibition by Z-VAD-FMK is less important, BOC-D-FMK is completely inactive against caspase-2. Caspase-3 like inhibitor (Z-DEVD-FMK) did not interfere highly with caspase-2 activity. Calpains inhibitor, E64d, is used as negative control. (B) Qco-VDVAD-dfp promotes survival of SD-cortical neuron culture. Qco-VDVAD-dfp was administrated to neurons at DIV6 at the initiation of SD for 24 hrs. Caspase-2 activity, ΔΨₘ loss, NA and PMP are determined by FLICA, JC-1, Hoechst 33342, and 7-AAD staining, respectively (n= 2). (C-E) Caspase-2 inhibition provides neuroprotection against neonatal in vivo ischemic brain injury : Effect of Q-VD-OPH and Qco-VDVAD-dfp on infarct volume measured 48 hrs after ischemia. The drug was given 5 minutes before the ischemic onset and consisted of a single intraperitoneal injection of the inhibitor (100 µg/10 g in 10 % DMSO, n=16 and 12 respectively). Control ischemic rats (n=15) were also studied. (C) Representative coronal sections at the level of the dorsal hippocampus (plate 21) and anterior commissure (plate 12) were obtained from ischemic control and Qco-VDVAD-dfp -treated animals and stained by cresyl-violet. Note the markedly reduced infarct in the treated-rat (animal with a 2 % infarct volume). The arrow indicates the presence and absence of an infarct in the same ischemic or Q-VDVAD-treated animal, respectively. Bar represents 130 pm. (D) Mean infarct volumes in the different groups. Data are mean ± SEM. Q-VD-OPH and Q-VD-VAD-OPH induced respectively a 44 and 74 % reduction (+++ = p<0.001, Kruskall-Wallis test). (E) Qco-VDVAD-dfp and Q-VD-OPH treatments provide two groups with animal displaying either high / total or low protection level. Single infarct volume data are plotted. Bold and thin horizontal bars represent the group median and mean, respectively. Note that 4 and 8 animals exhibited no infarct after Q-VD-OPH and Qco-VDVAD-dfp treatment, respectively.

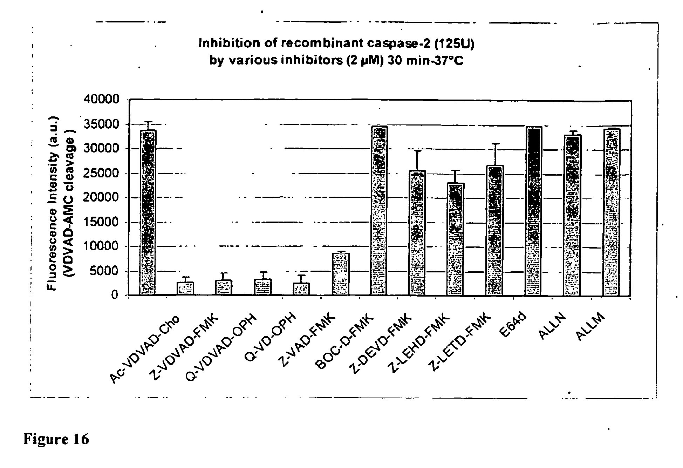

**Figure 16****. *In vitro* VDVAD-AMC cleavage by human recombinant caspase-2**
   The cleavage of 50µM VDVAD-AMC by recombinant human caspase-2 (125 U) was measured after 30 min at 37 °C prior to incubation with selective or pan-caspase inhibitors (2uM) (n ≥ 2). Caspase-2 cleavage activity is blocked by the prototype compound, Qco-VDVAD-dfp, as efficiently as specific caspase-2 inhibitors (Ac-VDVAD-Cho, Z-VDVAD-FMK) and pan-caspase inhibitor Q-VD-OPH. While cleavage inhibition by Z-VAD-FMK is less important, BOC-D-FMK is completely inactive against caspase-2. Other specific inhibitors for caspase-3 (Z-DEVD-FMK), caspase-9 (Z-LEHD-FMK) and caspase-8 (Z-LETD-FMK) did not interfere highly with caspase-2 activity. E64d, ALLN, ALLM that inhibit calpains are used as negative control.
**Ref.** **Figure 17****. Hypothetical model for pre-mitochondrial caspase-2 dependent pathway**
   We described a new intrinsic pathway in which pre-mitochondrial activation of caspase-2 is required to promote apoptosis in cortical neurons. Serum withdrawal is able to trigger apoptotic pathway, in which caspase-2 activation may mediate upstream control of Bax, a pro-apoptotic member of Bcl-2 family. Bax translocates and integrates into outer mitochondrial membrane to induce ΔΨₘ drop and to promote cytochrome c release in a caspase-2-dependent manner. Therefore caspase-2 inactivation abolishes also downstream events, like cytochrome c release-dependent activation of caspases-9 and caspase-3, nuclear morphological alterations, phosphatidyl serine exposure and terminal permeabilization of the plasma membrane. The exclusive cytoplasmic localization of active caspase-2 throughout long serum deprivation points into evidence a peculiar mechanism of activation.
**Ref.** **Figure 18****. Caspase-2 is involved during DNA-damage induced cell death and preceeds ΔΨₘ loss and PMP.**
   Dose-response of VP16 in absence or presence of caspase inhibitors: A and B showed the protective effect of caspase-2 like inhibition by specific caspase-2 inhibitor (VDVAD = Z-VDVAD-FMK). The effect of pan-caspase inhibitor (OPH=Q-VD-OPH) was also investigated. (A) n=3, JC-1/7AAD staining; (B) n=1, DioC6/PI.
**Ref.** **Figure 19****. Caspase-2 activation preceeds ΔΨₘ loss and subsequent caspase(s) activation.**
   (A) Left panel shows characteric apoptotic features for ΔΨₘ loss (JC-1) and nuclear alterations (Hoechst) in VP16 treated-Jurkat cells (10 µM, 7hrs). Right panel shows the effect of pan-caspase inhitor Q-VD-OPH or specific caspase-2 like (VDVAD=Z-VDVAD-FMK), caspase-3 like (DEVD=Z-DEVD-FMK), caspase-9 like (LEHD=Z-LEHD-FMK), caspase-8 like (LETD=Z-LETD-FMK) inhibitors, respectively on ΔΨₘ loss (JC-1), caspase-2 and caspase-3 activation (FLICAs), PMP, and nuclear alterations. All inhibitors are tested at 50 µM. (B) Quantitation, by flow cytometry of the effect of these inhibitors on ΔΨₘ loss (JC-1) and PMP (7AAD) (8 hrs). cycloheximide; BA= bongkrekic acid; DIDS= 4,4'-Diisothiocyanastilbeme-2,2'-disulfonic acid disodium salt; ActD= actinomycin D. (n=2-4)
**Ref.** **Figure 20****. Caspase-2 gene knock-down by a specific siRNA.**
   (A) Left and right panels show that hsiRNA C2 wt is able to decrease pro-caspase-2 protein pool in HeLa and Jurkat cells, respectively (Western Blot analysis; 11B4 clone for caspase-2 detection). (B) Transfection yield was checked *in cellula* by fluorescence detection (flow cytometry, FL-1) of siRNA-FITC: almost 100 % have incorporated siRNA (24 hrs).
**Ref.** **Figure 21****. Caspase-2 gene knock-down by a specific siRNA results in survival of VP16-treated Jurkat cells.**
   (A) Protective effect of (human) siRNA on VP16-treated Jurkats (7-8 hrs-10 µM) (n=3). Flow cytometry profiles showing that Z-VDVAD-FMK- and siRNA C2 wt-rescued cells have preserved morphology (Forward scatter) and that these cells are viable (7AAD exclusion). Lipo = lipectamine 2000 alone.
**Ref.** **Figure 22****. Sequence and structure of the sh-insert derived from the murine C2 siRNA sequence.**
   (A). The forward and the reverse oligonucleotides were designed to anneal between each other. Sequences in lower case represent the sens and antisens sequences of the siRNA directed against murin C2 mRNA. A BamH I and Xba I overhangs are added respectively at the 5' and 3' termini in order to improve the cloning in the pGE-1 vector.
   (B). The structure of the annealed shRNA illustrates the different functional regions of the shRNA Insert.
**Ref.** **Figure 23****. Level of expression of caspase-2 in 3T3 cells after transfection of shRNA -6 and shRNA-9 constructs. Western Blot** analysis of 3T3 total extracts (15 µg per lane) 24 or 48 hours after transfection with empty pGE-1 as a control (lane pGE-1) or with pGE-1 vector containing the shRNA insert (clones shRNA-6 and shRNA-9, lane shRNA6 and shRNA9). A control with lipofectamine alone has been done (lane lipo). NT lanes represent the non treated cells.
**Ref.** **Figure 24****. Sequence and structure of the sh-insert derived from the human C2 siRNA sequence.** (A).The forward and the reverse oligonucleotides were designed to anneal between each other. Sequences in lower case represent the sens and antisens sequences of the siRNA directed against human C2 mRNA. A BamH I and Xba I overhangs are added respectively at the 5' and 3' termini in order to improve the cloning in the pGE-1 vector. (B).The structure of the annealed shRNA illustrates the different functional regions of the shRNA Insert.
   Abbreviations: 7-AAD, 7-Amino Actinomycin D; 4-(2-Aminoethyl) benzenesulfonyl fluoride, AEBSF, Pefabloc; ANT, adenine nucleotide translocator; BA, bongkrekic acid; mClCCP, carbonylcyanide m-chlorophenylhydrazone; ΔΨₘ, mitochondrial transmembrane potential; FACS Fluorescence-Activated Cell Sorting; FLICA, Fluorochrome-Labeled Inhibitor of Caspase; FSC, forward scatter; FC, flow cytometry; FM, fluorescence microscopy; JC-1, 5,5',6,6'-tetrachloro-1,1', 3,3'-tetraethylbenzimidazolylcarbocyanine iodide; MFI, mean fluorescence intensity; PMT, photo-multiplicator tube; SD, serum deprivation; SSC, side scatter; MPTP, 1-methyl-4-phenyl-1,2,3,6-tetrahydropyridine; PS, phosphatidyl-serine; PTP, permeability transition pore; Quinoline-Val-Asp (OMe)-CH2-O-Ph, Q-VD-OPH ; SNP sodium nitroprusside; z-DEVD-fmk, N-benzyloxycarbonyl-Asp-Glu(Ome)-His-Asp(Ome)-fluoromethyl ketone ; z-VAD-fmk, N-benzyloxycarbonyl-Val-Ala-Asp(Ome)-fluoromethylketone.

### Ref. Example 1: Methods to identify checkpoint; multiparametric and dynamic analysis of neuronal apoptosis by fixed- and real-time cytofluorometry

Until recently, apoptosis and necrosis of neuronal cells have been mainly investigated by two types of approaches: the first group of (biochemical-) techniques evaluates late events of neuronal death generally by colorimetric evaluation of mitochondrial succinate dehydrogenase activity (MTT assay) or extracellular release of lactate dehydrogenase activity (LDH assay) (Johnson, 1995). These routine monoparametric quantitative techniques do not give informations concerning the mechanism of cell death and cannot be combined with the detection of other biochemical processes. More recently, some neuron-adaptated cell-fractionation protocols where published for the biochemical assessment of cytochrome c translocation by immunoblotting and caspases activation using fluorogenic substrates (Ethell and Green, 2002). Such recent methods give semi-quantitative informations on neuron populations but exclude multiparametric and real-time analysis. The second group of techniques use fluorescence microscopy (FM) read-out to detect organelles's modifications or apoptosis-related proteins. The majority of these FM studies are focused on late nuclear alterations including visualisation of chromatin morphology (Hoechst staining) and/or biochemical detection of DNA fragmentation (TUNEL assay). In few recent FM studies on neurons, immuno-localization of cytochrome c (in fixed cells), were reported, but in contrast to other fields of cell biology, a limited number of studies on neurons used the in situ detection of mitochondrial alterations and caspase activation. When applied to cultured primary neurons, FM-based analyses are time-consuming, laborious, and quantification is hampered by cellular body aggregates and overlapping neurite networks. In addition, photo-bleaching of sensitive fluorescent probes could lead to dramatic misleading interpretations and exclude real-time follow-up of early death-related events. Thus, to our knowledge, cell biology features of key apoptotic events have not been fully documented and ordered in primary neurons.

Flow cytometry (FC) offers a wide range of applications, and has become a major tool for cell biology and apoptosis. While extensively applied to primary blood cells and cancer cell lines, this technology remains strikingly underused in neurosciences and was generally limited to evidence the late loss of DNA content in fixed cells (Yan et al., 1999; Fall and Bennet, 1999). Lack of appropriate flow cytometry applications probably results from the assumptions that the required detachment of neurons from their substrate could alter plasma membrane integrity, destroy neurites and/or trigger anoikis, thus preventing reliable analysis of apoptosis. To overcome these (neuron-) specific limitations, we used a simple trypsinization method for the non-invasive detachment of primary neurons that maintains the integrity of neurons and preserves a high proportion of their neurites. Then, we developped a method which combines quantitative FC to detailled FM analyses, enables the co-detection of the decision, effector, early and late degradation phases of apoptosis. Using selected fluorescent (vital-) probes, this double read-out permits to detect - before (by FM) and after (by FC) trypsinization - mitochondrial transmembrane potential (ΔΨₘ) state, caspase activation *in situ*, surface exposure of phosphatidylserine residues, and loss of integrity of plasma membranes.

Using mouse primary cortical neurons induced to die by serum deprivation as a system model, it is demonstrated that FC is non-solely concordant with FM but is also a rapid, sensitive and quantitative technology to establish the chronological order of neuronal apoptotic events. In addition, the area of FC analysis is extended to innovative real-time monitoring of early neuronal ΔΨₘ modifications and plasma membrane permeabilization (PMP) within minutes after addition of mitochondrio-active compounds. Both fixed-time and real-time FC permit to overcome the limitations of FM and will help to document and develop the cellular biology of neuronal apoptosis.

### - Cytofluorometric analysis of living and dying primary neurons.

Primary cortical neurons isolated from embryonic day-14 mice can be maintained in life more than 10 days when cultured on polyethyleneimine-coated wells in an *ad hoc* medium containing a mixture of glucose, horse serum and foetal calf serum (Kawamoto and Barrett, 1986).

In these experimental conditions, fluorescence microscopy (FM) evaluation of both chromatin condensation (Hoechst 33342; blue fluorescence) and plasma membrane integrity using the cell-impermeant fluorescent DNA-intercalative 7-amino-actinomycin D (7-AAD; red fluorescence) indicates that serum-deprivation leads to progressive plasma membrane permeabilization (PMP) of cultured neurons (Fig. 1A). This PMP is a post-apoptotic event since it occurs only in shrinked neurons with condensed chromatin and dismantled neurites (Fig.1A). In contrast, when primary PMP (i.e. necrosis) is induced by low concentration of Triton, no cell shrinking nor chromatin condensation are detected (phase contrast and Hoechst fluorescence), but 7-AAD rapidly enters into neurons and labels nuclei (Fig 1B). To quantify unambigously neuronal shrink and PMP at any chosen time during cell death, conditions of trypsinization were established which permit to maintain neuron integrity as objectived by both the absence of staining with 7-AAD and stable neuronal-retention of the non-toxic CellTracker™ Green fluorescent dye (Fig.1B,C). Thus, neurons can be first labelled on their substrate and observed by FM, second safely trypsinized, and third immediately submitted to flow cytometry (FC) analysis (Fig. 1D-G). Beside intact 7-AAD negative (trypsinized-) neurons (88.4% +/- 7.6) in control samples , 47.1% (+/- 18.1) of 24-hour serum-deprived neurons present PMP (7-AAD+), correlating with microscopic observations and counting before trypsinization (Fig. 1E-G).

### - Detection of the degradation and effector phases in apoptotic primary cortical neurons.

FM based co-detection of PMP (7-AAD staining) and apoptosis-related phosphatidyl-serine (PS) exposure (FITC-conjugated annexin V; green fluorescence) indicates that in serum-deprived neurons three cell populations appear: a subset with both 7-AAD and FITC-annexin V staining (subset 2; Fig. 2A), and two subsets with either 7-AAD staining (subset 3) or FITC-annexin V staining (subset 1). Same subsets are also detected after trypsinization by FC, and kinetic follow-up shows that subset 1 precedes subset 2 which precedes subset 3 (Fig. 2B,C), thus leading to the conclusion that PS exposure occurs before PMP. The first detectable nuclear event is a significant progressive nuclear reduction (perimeter) which appears to precede neuron size modifications (Fig. 2D, E). This FC fixed-time analysis of neurons can be extented to caspases activation (Fig. 3). Indeed, *in situ* co-detection of caspase-3 like activity using a green Fluorescent Labeled Inhibitor of Caspase (FLICA, FAM-DEVD-FMK) and PMP (7-AAD staining) give similar results with FM (before trypsinization) and FC (after trypsinization) to show that a caspase-3 like activity is detectable before PMP (Fig. 3A,B). Similar results are obtained when FLICA-based detection of caspase-3 activity is replaced by *in situ* antibody-based detection of the activated caspase-3 (not shown). When added to neurons at the beginning of serum deprivation, both the new broad-spectrum inhibitor of caspase, Quinoline-Val-Asp (OMe)-CH2-O-Ph (Q-VD-OPH) (Melnikov et al., 2002) and the mitochondrial adenine nucleotide translocator (ANT) inhibitor, bongkrekic acid (BA), strongly prevent caspase activation, PMP and nuclear apoptosis (Figs. 3C,D). FC quantification indicates that in contrast to the pan serine-protease inhibitor 4-(2-Aminoethyl)-benzenesulfonyl fluoride (AEBSF, Pefabloc), Q-VD-ORH inhibits 95.3 +/- 5.6% of caspase-3 like activity and 93.9 +/- 3.8% of PMP (7-AAD) induced by serum deprivation (Fig. 3D). A non-trivial question is to determine, in a given cell death model, the hierarchy between caspase activation and PS exposure. *In situ* FM (before trypsinization) and FC (after trypsinization) co-detection of caspase-3 like activity using sulforhodamine-conjugated FLICA (red fluorescence) and PS exposure using FITC-annexin V (green fluorescence) are concordant to demonstrate that, after serum deprivation, caspase-3 activity precede PS exposure in primary neurons (Fig. 3E,F). It should be noted that simultaneous analysis of chromatin state (Hoechst; blue fluorescence) by FM indicated that early caspase-3 activity is temporally associated with a first step of nuclear condensation (stage-I according to Susin's classification; Susin et al., 1999), although (Fig. 3E, 4E) terminal nucleus fragmentation into discrete apoptotic bodies (Stage-II morphology, Susin et al., 1999) occurs after the beginning of PS exposure. Intriguingly, both the benchmark pan caspase-inhibitor z-VAD-fmk and the more restricted caspase-3 like inhibitor z-DEVD-fmk strongly inhibit caspase-3 activation, but not the degradation phase (i.e. PS exposure, nuclear condensation and PMP) of neuronal apoptosis (Fig. 3D), thus indicating that caspase-3 related activity is not essential for neuron death in these experimental conditions. In contrast, *in situ* co-detection of chromatin state (Hoechst), and caspase-9 like activity using a green Fluorescent Labeled Inhibitor of Caspase (FLICA, FAM-LEHD-FMK) in the presence or absence of the caspase-9 inhibitor. z-LEHD-fmk reveals that abolition of caspase-9 like activity leads to an intermediate phenotype of nuclear apoptosis in which most nuclei are arrested at the first step of nuclear condensation (stage-I; Fig. 3G). Moreover, both FM and FC analysis are concordant to show that caspase-9 inhibition abolishes PS exposure and PMP (Fig. 3G,H). Thus, since BA prevents caspase-9 like activation (Fig.3H), the double read-out approach strongly suggests that the execution point of caspase-9 in this experimental model is downstream mitochondria and upstream PS exposure and stage-II nuclear apoptosis.

### - Detection of the mitochondrial/decision phase of neuronal apoptosis.

Staining of cultured primary neurons with the ΔΨₘ -sensitive dye JC-1 followed by FM analysis reveals a progressive ΔΨₘ loss. Thus, before serum deprivation, mitochondria from neurons possess a high ΔΨₘ (orange JC-1 fluorescence; Fig. 4A), whereas mitochondria from 8-24 hours serum deprived neurons have a low ΔΨₘ (green JC-1 fluorescence; Fig. 4A). The ΔΨₘ loss progressed heterogeneously without any appearent geographical hierarchy, giving rise to a transient intermediate phenotype in which heterogeneity is detectable in the same neuron (Fig. 4A; Dec). This suggest that at least in this experimental system there is no simultaneous coordinated ΔΨₘ loss, but rather a progressive transmission of the collapsing signal from mitochondria to mitochondria. Full ΔΨₘ disruption is observed before any sign of nuclear apoptosis as objectived by Hoechst staining (Fig. 4A; blue fluorescence). As expected, FM and FC-based co-quantitation of ΔΨₘ loss (JC-1) and PMP (7-AAD staining) are concordant to demonstrate that ΔΨₘ loss is inhibited by BA and precede PMP in serum deprived neurons (Fig. 4B-E). Kinetic experiments based on the co-detection of ΔΨₘ (using the ΔΨₘ -sensitive dye CMX-Ros) and caspase-3 like activity (FLICA, FAM-DEVD-FMK), suggest that ΔΨₘ loss precedes caspase-3 activation (not shown). Accordingly, inhibition of caspase-3 activation by z-DEVD-fmk has no effect on SD-induced ΔΨₘ loss (Fig. 4E).

### - Real-time detection of ΔΨₘ

The early involvement of mitochondria in neuronal apoptosis requires the monitoring of rapid ΔΨₘ responses to drug exposure. Real-time detection of ΔΨₘ by FM can skew analyses since repetitive acquisitions provoke a dramatic photobleaching of the probe (detected as a drop in JC-1 orange fluorescence), which could be wrongly attributed to apoptosis-related ΔΨₘ loss (Fig. 5A, B). To overcome this instrumental drawback, a real-time FC approach was developped in which, in contrast to the fixed-time FC protocol, neurons are first trypsinized and second labelled to detect ΔΨₘ (JC-1) and PMP (7-AAD) over time (Fig. 5C). In these conditions, FM observations reveal that trypsinized neurons do not present PMP and maintain high ΔΨₘ up to 3 hours (Fig. 5C). It should be noted that no signs of anoikis are detectable during the first 5 hours post-trypsinization. FC recording for 20 minutes confirms that trypsinized neurons still have a stable elevated ΔΨₘ and are impermeant to 7-AAD, i.e. keep an intact plasma membrane (Fig. 5D2-3). Addition of the respiratory chain uncoupler, carbonyl cyanide m-chlorophenylhydrazone (mClCCP), to non-trypsinised neurons induces ΔΨₘ disruption (Fig. 5D-1). Real-time FC monitoring reveals that ΔΨₘ loss of neuronal population is maximal after 2 minutes of treatment with mClCCP (Fig. 5D-2,3). FM co-detection of PMP (7-AAD) and ΔΨₘ (JC-1) of non-trypsinized neuron cultures treated with the mitochondrial toxin 1-methyl-4-phenyl-1,2,3,6-tetrahydropyridine (MPTP), indicates that after 45 minutes most neurons are ΔΨₘ low without any sign of PMP (Fig. 6A-1). In contrast, cortical neurons treated or not with the nitric-oxide inducer SNP maintain an elevated ΔΨₘ (Fig. 6). As expected ethanol induces a rapid PMP as objectived by massive 7-AAD incorporation in cultured neurons (Fig. 6A-1). When real-time FC is applied to simultaneous evaluation of PMP, ΔΨₘ, cell size and granularity of cortical neurons, this technique indicates that, after 15 minutes, 49.6% (+/- 8.2; n=4) of MPTP-treated neurons are ΔΨₘ low, whereas 16.2% (+/- 1.2) of untreated neurons and 15.0% (+/- 6.2) SNP-treated neurons are ΔΨₘ low. Real-time FC reveals that, in contrast to MPTP and SNP, ethanol treatment induces primary necrosis. Indeed, ethanol triggers a very rapid PMP (98% after 5 minutes) which precedes ΔΨₘ loss (75% after 5 minutes) (Fig. 6). Interestingly, MPTP-induced ΔΨₘ loss is heteregeneous since neurons which undergo rapid ΔΨₘ drop present a significant granularity increase, whereas neurons which undergo a slight ΔΨₘ reduction do not present morphological modifications (Fig. 6).

Taken together, these results show that real-time FC analysis is a simple approach to quantitatively follow up short term PMP events and ΔΨₘ modifications on a per-neuron basis.

Using serum-deprived mouse primary cortical neurons as a system model it is then shown that: 1) neuronal samples can be multi-labeled with apoptosis-related probes and successively analysed by FM, safely detached from their support and quantitatively studied by FC without fixation, 2) kinetic and pharmacologic informations obtained with this double read-out methodology permits to describe and unambiguously order the main phases (decision, execution and degradation) of neuronal apoptosis, 3) neuron can also be first detached from their support, then labelled with vital probes and analysed by real-time FC for 3 hours, thus offering the possibility to asses short term events of neuronal death including discrimination between primary necrosis (i.e. when PMP precede ΔΨₘ loss) and apoptosis-related cell-responses to a given stimulus.

FC offers some specific advantages (Table 1). First, whatever the initial level of aggregation of neurons in culture, FC permits to get rapidely a representative quantitation of apoptosis and related events on a high number of neurons (40,000 per sample in this study). Second, FC can detect intracellular probes with low levels of fluorescence that could be hardly evidenced by FM. This advantage can be attributed to the better ability of the cytometer photomultiplier tubes (FC) to discern weakly fluorescent cells, comparatively to charge coupled device (CCD) camera (FM). Third, FC also overcome problems classically induced during FM observations including probes photobleaching (as it is the case for ΔΨₘ detection by JC-1), cell damage induced by long epifluorescence illumination and/or photothermal effects. For instance, JC-1 photobleaching is minimal with FC because of the weak neuron irradiation (15 milli-Watts, monochromatic wavelength) in comparison to FM (5-Watts, polychromatic wavelengths) and the extremely short (and unique) cell passage through the laser beam. Fourth, real-time FC authorizes the quantitative analysis of very short term plasma membrane and mitochondrial inner membrane modifications within minutes following addition of any neuro-active drug. Fifth, multiparametric analysis may be enlarged by the use of more powerful cytometers that can investigate up to 14 individual parameters.

It is also demonstrated that SD neurons undergo an apoptotic process that obeys the following rules (Figure 7). First, SD-neurons manifest signs of ΔΨₘ dissipation through an ANT related dependant process. Second, ΔΨₘ dissipation occurs upstream caspases 3 and 9 activation. Third, PS exposure and full nuclear condensation (stage-II) are subordinate to a caspase-9 like activity but do not depend on caspases-3-like activity. Paradoxically, Z-VAD.fmk-treated 24 h SD-neurons do not present caspase-3-like activity but undergo PS exposure, stage-II nuclear apoptosis and final PMP, whereas all this events are fully blocked by the third generation pan-caspase inhibitor Q-VD-OPH. Hence, the above results reveal an unusal mitochondrio-dependant caspase pathway which is activated in primary cortical neurons during apoptosis induced by serum withdrawal.

This cytofluorometric technology was also used to investigate apoptosis dynamics of neurons in response to other stimuli, including ceramide, β-amyloid peptides, 3-nitropropionic acid, glutamate and viral proteins. Analysis was also extended to detect the activation of other caspases involved in neuronal apoptosis. These cytofluorometric analyses can also enable better characterization of still poorly known types of death, such as the non-apoptotic form of programmed death of cortical, striatal and hippocampal primary neurons treated by substance P, and make it possible to differentiate between necrosis-like deaths and apoptosis in models where both coexist, such as ischemic injury.

Hence, the technologies developed according to the invention are powerful to investigate the cell biology of neuronal apoptosis and provide a multiparametric quantitative tool for the screening and characterization of neurotoxic and neuroprotective compounds.

### Experimental procedures

### Isolation and culture of cortical neurons

Primary cortical neurons were isolated from neocortices of embryonic day-14 Swiss mice (Janvier, Le Genest-St-Isle, France). Neurons were plated at a density of 7.10⁵ live cells per cm² in 500 µl of Eagle's Basal Medium (EBM, Eurobio, Les Ulis, France) supplemented with 5% horse serum (HS, Eurobio) and 2.5% fetal calf serum (FCS, Eurobio) onto 24 well-plates (Sarstedt, Orsay, France) or Lab-Tek chambered coverglasses (Nalge Nunc Internationnal, Naperville, IL, USA) coated with polyethylenimine (PEI, 1 mg/mL, Sigma, St Quentin Fallavier, France). After 2 days, the culture medium was replaced with N5 medium (Kawamoto and Barrett, 1986) containing 180 mg/L glucose, 5% HS and 1% FCS, and 3 µM of cytosine β-D-arabinofuranoside (Ara C, Sigma) and 1 µM of 5-methyl-10, 11-dihydro-5H-dibenzocyclohepten-5,10-imine maleate (MK-801, Research Biochemicals International) (Knusel et al., 1990) and changed daily. Apoptosis was induced in 5 days-old cultures by serum withdrawal (Macleod el al., 2001). Purety of culture (> 95%) was assessed with an anti-Microtubule Associated Protein 2 monoclonal antibody (MAP-2, Sigma) and anti-Glial Fibrillary Acidic Protein polyclonal antibody (GFAP, Dako).

### Cortical neurons trypsinization

Enzymatic detachment of neurons was performed after one careful washing in serum-free N5 medium and incubation with 250 µl of 37°C Trypsin-EDTA (Gibco BRL, UK) for 15 min at 37°C. Cell detachment was performed by 5 gentle flushes, using 1000 µl tips (Gilson). The remaining neuron aggregates were dissociated through a 200 µl tip by 10 careful flushes in 500 µl N5 medium. For the validation of the trypsinization procedure, adherent neurons were stained by 10 µM CellTracker Green™ (Molecular Probes, Eugene, OR) for 15 min at 37°C, washed in N5 medium, and submitted to trypsinization. Neurons analysis was performed by flow cytometry (FL-1 channel) an microscopy (BP 480/40 for excitation and BP 527/30 for emission). Triton X-100 (Sigma) treatment (0.02%) was used as positive control for plasma membrane disruption.

### Instrumentation

Fluorescence-Activated Cell Sorting was performed using a 3-color FACSCalibur cytometer equipped with a 15 mW air-cooled 488 nm argon laser (Becton Dickinson, San Jose, CA). For each sample, data from 40,000 neurons were registrated, and analysed with the CellQuest Pro^{™} software (Becton Dickinson). The sample flow rate was setted to 12 µl +/- 3 µl/min for real-time analyses, and to 60 µl +/- 3 µl/min for fixed-time experiments. Fluorescence microscopy (FM) was performed with a DM IRB inverted fluorescence microscope (Leica, Rueil-Malmaison, France) equipped with a 100 W mercury short arc lamp and a X 40 N PLAN L objective or a water immersion X 100 N PLAN objective (Leica, Wetzlar, Germany). Pictures were acquired at a resolution of 1300 x 1030 pixels with a CCD color camera (Leica DC 300F, Leica, France) and controled by the Leica QFluoro software (Leica Microsystem AG, Switzlerland). Data were stored for off-line analysis with IM1000 software (Leica Microsystem AG) to be carried out using the Leica QFluoro software.

### Detection of the degradation phase of apoptosis through incorporation of 7-Amino Actinomycin D

The loss of the plasma membrane integrity was detected through the increased permeability to 7-Amino Actinomycin D (7-AAD, Sigma) (Schmid et al., 1992; Carpenter et al., 1997; Lecoeur et al., 2002). 20 µg/ml 7-AAD were added to cultured neurons for 15 min at 37°C. FM analysis was performed through a 100 ms excitation using a BP 515-560 filter and 7-AAD fluorescence was detected through a LP 590 long-pass filter. Cells were trypsinized and immediately analysed on the flow cytometer (Fl-3 channel, λ> 650 nm, PMT = 333). Apoptotic bodies/debris were discarded from analysis as described for cells growing in suspension (Lecoeur et al., 1997).

### Detection of early and late degradation phases using FITC-annexin V and 7-AAD

Phosphatidylserine exposure (PS) to the outer layer of the plasma membrane was detected through the fixation of FITC conjugated- annexin V (Apoptosis detection KIT, R&D System). 20 µg/ml 7-AAD and 1X annexin V were added into 200 µl of Ca²⁺ -enriched buffer (Apoptosis detection KIT) for 20 min at RT. For FM experiments, annexin V-FITC was excited through the BP 480/40 filter and the emitted light was collected using the BP 527/30 filter. FC detection of FITC- annexin V fluorescence was performed in the F1-1 channel (530 +/- 15 nm), and analysed in linear amplifier mode, (PMT voltage = 867, amplification gain = 9.00). Spectral overlap was avoided by adjusting compensation network as follows: FL2 - 22.9% FL1 and FL2 - 41.7% FL3.

### Combined detection of the effector and degradation phases using FLICA, annexin V and 7-AAD

Activated caspase-3 and caspase-9 were detected using FAM-DEVD-FMK and FAM-LEHD-FMK, both Fluorochrome Labeled Inhibitors of Caspase (FLICA) (CaspaTag^{™} fluorescein Caspase Activity Kits, Intergen, NY) (Lecoeur et al., 2002; Smolewski et al., 2002). Neurons were incubated with 1/150 of the DMSO stock solution of the FLICA for 1 hr at 37°C. 7-AAD and Hoechst were added during the last 15 min. Then neurons were washed three times in washing buffer (CaspaTag^{™} Kit). For FM imaging, FLICAs were excited through the BP 480/40 filter and the emitted light was collected through the BP 527/30 filter. For FC analysis, FLICA fluorescence was collected through the F1-1 channel (PMT voltage = 501, compensation network: FL1 - 7.8% FL2, FL2 - 40.8% FL1 and FL2 - 45.4% FL3). Cleaved caspase-3 was evidenced in cellula by immunodetection using Phycoerythrin (PE)-conjugated polyclonal antibodies (Beckton Dickinson). Neurons were stained by 7-AAD, trypsinized and fixed in PBS containing 1% PFA and 20 µg/ml Actinomycin D (AD) for 20 min. Then, neurons were resuspenped in 100 µM PBS, 1% BSA, 20 µg/ml AD, 0.05% saponin Quillaja bark (Sigma) and 20 µl of the anti-caspase-3 antibodies for 30 min at RT (Lecoeur et al., 2001). After washings in PBS, PE-related fluorescence was analysed on the cytometer (Fl-2 channel). Z-val-Ala-Asp(OMe)-FMK (Z-VAD-FMK), Quinoline-Val-Asp (OMe)-CH2-O-Ph (Q-VD-OPH), Z-DEVD-FMK (Z-Leu-Glu(OMe)-His-Asp(OMe)-fmk, ICN) and Z-LEHD-FMK (Z-Asp(OMe)-Glu(OMe)-Val-Asp(OMe)-FMK, all purchased from ICN (Orsay, France), and 4-(2-Aminoethyl)-benzenesulfonyl fluoride (AEBSF, Pefabloc SC, Roche, Meylan, France) were added at 100 µM at the initiation of serum deprivation. Sulforhodamine-DEVD-FMK, (CaspaTag^{™} Red Activity Kit) permitted to detect activated caspase-3 and FITC-Annexin V. Neurons were incubated with 1/900 of the DMSO stock solution of the FLICA, and 1X FITC-annexin V in 200 µl of annexin-buffer for 30 min at 37°C. Then neurons were washed three times in a buffer composed of 50% washing buffer and 50% annexin-buffer. Caspase-3 activity was detected in the F1-2 channel (585 +/- 21 nm). For FM, FLICA was excited through the BP515-560 filter and its fluorescence was collected through the LP590 long pass emission filter.

### Fixed-time detection of the decision phase of apoptosis using JC-1 and 7-AAD.

Mitochondrial transmembrane potential (ΔΨₘ) was assessed by 5,5',6,6'-tetracholoro-1,1,3,3'-tetraethylbenzimidazolyl carbocyanine iodide (JC-1, Molecular Probes, Eugene, OR) incorporation. Neurons were co-stained with 1 µM JC-1 and 7-AAD for 15 min at 37°C. JC-1 monomers were detected by FC in the Fl-1 channel (PMT voltage = 644). J-aggregates were detected through the F1-2 channel (PMT voltage = 451) (Reers et al., 1991). The PMT voltage for 7-AAD detection was of 326. Compensation network: FL1 - 0.0% FL2, FL2 - 22.9% FL1, FL2 - 41.7% FL3, and FL3 - 0.7% FL2. For FM analysis, green and orange fluorescences were simultaneously recorded after 1.2 s excitation (BP 450-490 excitation / LP 515 long-pass emission filters). Photobleaching was avoided by attenuation of the irradiation to 5% of the initial incident light by a N20 neutral density filter. Bongkrekic Acid (BIOMOL,) was tested at 25 µM.

### Real- time detection of mitochondrial transmembrane potential ΔΨₘ) and neuronal morphology

Real-time experiments were performed on 5-days old cultured neurons right after trypsinization. Neurons were resuspended in N5 medium, adjusted to 0.7 10⁶ cells / ml and loaded with 800 nM JC-1 for 15 min at 37°C. Then, samples were diluted to 1/8 in N5 medium and 20 µg/ml 7-AAD were added. Basal morphology and ΔΨₘ and membrane permeability were registrated for 5 minutes, and drugs were added; 100 µM Carbonyl cyanide m-chlorophenylhydrazone (mClCCP, Sigma), 1 µM 1-methyl-4-phenyl-1,2,3,6-tetrahydropyridine (MPTP, Sigma), and 0.6 µM Sodium Nitroprusside (SNP, Sigma). MPTP is a mitochondrial complex-I toxin and an apoptosis inducer used *in vivo* to reproduce Parkinsonism in mice and primates (Speciale, 2002). Variations of every parameter were recorded for the following 15 min. Curves were drawn using the Microsoft Excel software.

### Nucleus staining by Hoechst 33342 and nuclear perimeter measurements

Neurons were incubated for 15 min with 1 µM Hoechst 33342 (Ho 342, Sigma) and analysed by FM (5 milliseconds exposure (BP 340-380 excitation filter/ LP 425 long-pass filter). The perimeter of nuclei was measured by creating individual regions of interest processing masks using the Leica Q Fluoro software, as expressed in arbitrary units.

### Statistical analysis

Statistics were performed using the Microsoft Excel software. Correlations were calculated by linear regression analysis. For each analysis, R² is indicated. Unpaired Student's t test was performed to compare percentages of cells in the different apoptosis stages. A p value < 0.05 was considered as significant.

### EXAMPLE 2: Caspase-2 inhibition/silencing in neuronal in vitro and in vivo cell death

Pan-caspase inhibition promotes survival of primary cortical neurons cultures induced to die by serum deprivation

During neuronal development and pathology, neurons that fail to find appropriate trophic support and sources of target-derived trophic factors undergo apoptotic cell death. Serum-deprivation (SD) of primary cortical neurons, an *in vitro* model for acute neuronal injury, leads to apoptotic cell death. Studying the hierarchy and temporal ordering of apoptotic hallmarks during SD, an intrinsic-like pathway has been described in which mitochondrial membrane potential (ΔΨₘ) disruption occurred upstream of nuclear apoptosis (NA) (condensation/fragmentation into apoptotic bodies), of phosphatidylserine (PS) exposure to outer plasma membrane leaflet, and terminal permeabilisation of the plasma membrane (PMP) Said results demonstrate the time-responses for such apoptotic hallmarks throughout 50 hrs SD (Figure 8A). For clarity, kinetics of appearance of neurons with low ΔΨₘ, NA, PS ecto-exposition or PMP reflect all the intermediates subsets with progressive alterations. In these experimental conditions, most neurons engage at the same time in each process.

Because of the critical role of caspases in several paradigms of apoptosis, caspases requirement has been evaluated during SD in cortical neurons. When added at the initiation of serum withdrawal, SD-neurons are mostly rescued by continuous treatment with Quinoline-Val-Asp(OMe)-CH₂-O-Ph (Q-VD-OPH), a new generation of broad spectrum caspase inhibitor, resulting in high preservation of ΔΨₘ and nuclear morphology, intact plasma membrane as well as absence of PS exposure (Figure 8B). In contrast, neither Z-VAD-FMK nor BOC-D-FMK (BOC-D) is able to delay or to abrogate SD-associated cell death (Figure 1B). It should be noted that nuclear morphology and both neurite integrity and neuritic network appear preserved enough in neurons rescued at 24 hrs by Q-VD-OPH (Figure 8C). Nevertheless, their soma is slightly smaller. Using specific fluorescent substrates, *in cellula* caspase-2 like, caspase-3 like, caspase-8 like and caspase-9 like activities were detected at 24 hr-SD (Figure 8D). The low level of caspase-8 like activation during SD suggests that extrinsic pathway is not preponderant in this model. All these caspase activities are completely inactivated by co-treatment with Q-VD-OPH (Figure 8D). Investigations were carried out to determine whether survival may be improved by Q-VD-OPH during challenges by other unrelated caspase-dependent neurodegenerative stimuli: Ca²⁺ ionophore ionomycin (excitotoxicity), the NO-donor sodium nitroprusside (SNP), β-amyloid (25-35) peptide (βA) and mitochondrial toxins such as 1-methyl-4-phenyl-1,2,3,6-tetrahydropyridine (MPTP) or the 3-nitropropionic acid (3-NPA). These drugs induce apoptosis (NA and PMP were monitored), but concomitant treatment with either Z-VAD-FMK or Q-VD-OPH fails to provide protection, except for β-amyloid, that is in agreement with previous report (Figure 8E). These findings reinforce the specific involvement of caspases during SD in cortical neurons.

To ensure the importance of primary caspase activation in the tested system model, pharmacological inhibition of various signaling and metabolic pathways was performed using the following compound families (Table I): mitochondria- and permeability transition pore (PTP)-targeting agents, mitochondrial calcium uptake modulator, cytoplasmic calcium chelator, inhibitors of proteases (calpains, serine proteases, proteasome or lysosomal cathepsins), cell cycle inhibitors, inhibitors of kinases and phosphatases involved in signal transduction pathways, agents interfering with endocytosis and autophagy processes, antioxidants, inhibitor of protein nuclear export. Almost all tested compounds fail to prevent cell death evoked by SD. As, pleiotropic agents cycloheximide and actinomycin D, that inhibited traduction and translation, promote survival of cortical neuron subjected to SD (Table I).

### Pre-mitochondrial caspase-2 activity is required for cortical neurons apoptotic cell death induced by serum-deprivation

The fact that an early event, such as ΔΨₘ loss, is prevented by Q-VD-OPH raises the questions of both the importance of (pre-mitochondrial) caspase(s) in the present model and the specificity of Q-VD-OPH. In order to identify the more proximal caspase activity responsible for cell death in SD model, a panel of more selective caspase inhibitors was used and their impact analysed on several parameters of cell death (Figure 9A): Z-DEVD-FMK, Z-LEHD-FMK, Z-VDVAD-FMK and Z-LETD-FMK that respectively inhibit caspase-3, -9, -2, and -8 like activities. It appears that only Z-VDVAD-FMK, an efficient caspase-2 like activity inhibitor (Figure 9 D), is able to both abolish loss of ΔΨₘ loss as well as others hallmarks of apoptosis (NA, PMP, PS exposure) and protect neurons against death (Figures 9A and 9B). To better characterize the inhibitory profile of Q-VD-OPH and Z-VDVAD-FMK, a best pattern for neuroprotection was determined. Apoptosis is inhibited by Q-VD-OPH and Z-VDVAD-FMK in a concentration dependent-manner, reinforcing that the caspase cascade is activated during SD in cortical neurons (Figure 10). Considering the high-density of the culture (7x10⁵ per cm²), the higher protective effect is provided by 100 µM of each inhibitor, that is the concentration used in this study. Addition of these inhibitors (100 µM) at the initiation of SD is the best pattern for Q-VD-OPH- or Z-VDVAD-FMK-induced neuroprotection, since treatments with inhibitors delayed for 2-6 hrs post-serum withdrawal are less efficient (Supplementary Material; Figure 10). Moreover caspase-2 like activation is detected from 2 hrs of SD and precedes both first signs of ΔΨₘ drop (8 hrs) and further nuclear alterations (Figure 9C). Altogether, these findings showthat a pre-mitochondrial caspase-2 like activity is the most proximal caspase activity required for SD-induced apoptosis in cortical neurons. Caspase-2 like activity is abolished by Z-VDVAD-FMK but not by Z-DEVD-FMK, Z-LEHD-FMK or Z-LETD-FMK (Figure 9D). In contrast caspase-3 like and caspase-9 like activities are inhibited by Z-VDVAD-FMK thus demonstrating that caspase-2 like activation is upstream both caspase-3 like and caspase-9 like activities (Figure 9D). While respectively abolishing caspase-3 like and caspase-8 like activities (Figure 9D), Z-DEVD-FMK and Z-LETD-FMK failed to protect neurons from SD (Figures 9A and 9B), thus indicating that caspase-3 related activity and that the recruitment of caspase-8 are not essential for neuronal degeneration. Furthermore the caspase-8 inhibitor failed also to block the activation of caspases-2,-3 or -9 (Figure 9D). The caspase-9 inhibitor, Z-LEHD-FMK does not impair □□ₘ drop whereas it delays and prevents apoptotic bodies formation but not stage I-condensation (NA) PS exposure and PMP (Figures 9A and 9B).

These data show that caspase-2 acts upstream of MMP and that caspase-9 acts downstream MMP during SD.

In order to confirm this assessment, genetic proof for caspase-2 activity-mediated apoptosis induced by SD has been investigated. Sequence analysis of murine caspase-2 led to the design of specific small interfering RNA (siRNA C2 wt) directed against murine caspase-2, that induces specifically knock-down of caspase-2 expression, as assessed by RT-PCR and Western blotting (Figure 11A). As a control, an irrelevant siRNA with 4 mutations (siRNA C2m) was designed.
siRNA C2 wt duplex is:
   SEQ ID N°1 5'-caccuccuagagaaggacadTdT- 3'
   SEQ ID N°2 5'-uguccuucucuaggaggugdTdT- 3'
siRNA C2 m duplex is:
   SEQ ID N°3 5'-caucuacucgagacggacadTdT-3'
   SEQ ID N°4 5'-uguccgucucgaguagaugdTdT-3'

*In situ* antibody-based detection confirms high gene silencing of murine caspase-2 since siRNA C2 wt decreases caspase-2 expression in all neurons (Figure 11B). The extinction is maximal at 24 hrs post-transfection with progressive recovery of caspase-2 expression at 72 hrs (Figure 11B). Strikingly, knock-down of caspase-2 by siRNA C2 wt results in survival of cortical neurons after SD, as assessed *in cellula* by caspase-2 inactivation (Figures 11C and 11D) as well as preservation of ΔΨₘ, NA, PS symmetry, plasma membrane integrity and neuritic network (Figures 11C-E). In sharp contrast, control siRNA C2m prevents neither gene/protein expression (Figures 11A) nor the appearance of these apoptosis hallmarks (Figures 3C and 3D). Moreover the impact of caspase-2 inhibition or extinction on cell survival is specific of SD since ionomycin-treated neurons are not protected against cell death (figures 11D and 11E). Thus, treatment with this Ca ²⁺ ionophore is a useful caspase-2 independent control to probe the specificity of siRNA C2 wt since caspase-2 is not activated (see below) and Z-VDVAD-FMK or siRNA C2 wt provides no protective effect (Figures 11D and 11E).

These results demonstrate that caspase-2 activation' is a crucial pre-mitochondrial checkpoint in this model.

### Caspase-2 controls both cytochrome c release and Bax translocation into mitochondria

Investigations were performed to determine whether a MMP-dependent event, such as cytochrome c release is prevented or not by caspase-2 inhibition or knock-down. SD triggers cytoplasmic cytochrome c release from mitochondria that is efficiently blocked by Q-VD-OPH, Z-VDVAD-FMK and siRNA C2 wt (Figure 12A). Similarly, Q-VD-OPH, Z-VDVAD-FMK and siRNA C2 wt abolish caspase-2 activation and prevent downstream cytochrome c release-dependent activation of caspases-9 and caspase-3 (Figures 12B and 12C). Cell death induced by ionomycin is independent of caspase-2 activation in cortical neurons (Figure 12B), that agreed with the absence of protective effect on other hallmarks of apoptosis by Z-VDVAD-FMK and siRNA C2 wt (Figures 12D and 12E). It should be noted that the inhibition of more distal caspases as, caspase-9 (by Z-LEHD-FMK) and caspase-3 (by Z-DEVD-FMK) could not prevent cytochrome c release (Figure 12A) whereas Z-LEHD-FMK may delay later apoptotic features, as observed by higher frequency of blockage in a preliminary stage of nuclear condensation (stage I) (Figure 12A). Altogether with the fact that Z-LEHD-FMK does not impaired ΔΨₘ drop whereas it prevents caspase-9 activation and terminal features of apoptosis, i.e, PS exposure, NA and PMP (Figures 9A, 9B and 9D), these results support the formation of the classic apoptosome implying cytochrome c, caspase-9 and the subsequent caspase-3 activation.

The role of Bax relatively to caspase-2 was then studied, since this pro-apoptotic protein of the Bcl-2 family, is required during neuronal development and may be also critical to promote mitochondrial cytochrome c release and cell death in neurons after trophic factor. *In situ* antibody-based detection of Bax was performed in SD-neurons and shows Bax translocation from cytosol (diffuse pattern) into mitochondria-like compartments (punctuated) (Figures 12D), demonstrating that Bax may also participate in initiation of cell death. Importantly, positioning caspase-2 activation versus Bax translocation is crucial to understand if (i) Bax translocation is dependent on caspase-2; (ii) if caspase-2 activity is dependent on Bax; (iii) if both are independently involved in pre-mitochondrial control of SD-induced cell death.

It was observed that Bax remains diffuse in the cytosol of SD-neurons treated by Z-VDVAD-FMK, thus suggesting that caspase-2 may control Bax translocation to promote cell death (Figure 13A). On the contrary, Z-LEHD-FMK that acts on caspase-9, the more close caspase activated downstream of mitochondria, does not prevent mitochondrial Bax relocation. In agreement, treatment with Z-VDVAD-FMK, Q-VD-OPH or caspase-2 knock-down by siRNA C2 wt impair Bax translocation to mitochondria (Figure 12D), confirming that caspase-2 may exert an upstream control of Bax to promote cell death. To better characterize the putative relationship between Bax and caspase-2, primary cortical neurons induced to die by SD were treated with the chloride channel inhibitor furosemide. Indeed Bax translocation seems to require pH and ionic strength-sensitive conformational changes, and furosemide has been shown to reduce Bax translocation within cells treated with staurosporine, Tumor Necrosis Factor-α or etoposide. By interfering with Bax translocation (Figures 13A and 13B), furosemide (that may act at the level or upstream of Bax) reduces hallmarks of apoptosis, (*i.e*, ΔΨₘ loss, cytochrome c release, NA, PMP) in SD-neurons (Figures 13C). Moreover, fine kinetic observations reveal that partial Bax relocation into mitochondria occur at 5 hrs SD (nearly concomitant with caspase-2 activation; Figure 9C), before ΔΨₘ loss at 8 hrs and cytochrome c release from mitochondria at 15 hrs (data not shown), suggesting that Bax mediates MMP in SD paradigm. Importantly, although furosemide blocks Bax translocation, it partially prevents mitochondrial Bax relocation upon SD but does not impair caspase-2 activity (Figure 13B). It should be remarked that furosemide provides only a partial protection compared to Z-VDVAD-FMK or siRNA C2 wt, that may be attributable to the dose limitation (more than 3 mM is toxic for cortical neurons) and the fact that furosemide is not a direct Bax-interfering agent.

Caspase-2 activity is non-nuclear and remains diffuse in the soma and neurites of SD-neurons as well as in those treated with furosemide, suggesting no organelle-specific caspase-2 activity. This observation is crucial since Z-VDVAD-FMK or siRNA C2 wt impair both Bax translocation and caspase-2 activity (Figure 13B). Altogether, these data suggest an upstream caspase-2-dependent redistribution of Bax from cytosol to mitochondria, which in turn initiates a linear sequence of events in which ΔΨₘ loss, downstream cytochrome c release-dependent activation of caspase-9 and caspase-3, NA, PS exposure and final PMP occur. However, a putative direct or indirect Bax-independent action of caspase-2 on mitochondrial membrane in neurons cannot be excluded, as suggested in cell-free systems.

### SD-induced Bax cleavage is dependent on cytoplasmic caspase-2 but is calpain-independent

In order to establish precisely the connection between Bax and caspase-2, the expression of caspase-2 and Bax in SD-neurons was checked at mRNA and protein level and the search was focused to precise cellular localization of active caspase-2 throughout SD.

Concerning Bax, no mRNA up/down-regulation (Figure 14A) nor p22 Bax protein content increase are detected following 24 hr-SD (Figure 14B). Strikingly, in addition to the native full-length p22 Bax, throughout 24 hr-SD, the progressive appearance of a second band corresponding to a protein of 18 kDa was observed when detected by Western Blotting using the polyclonal antibody (Δ21) raised against whole mouse Bax α deleted for the carboxy terminal 21 amino acids (see the time-course in Figure 14B). A comparative immunoblotting of the p22 and p18 Bax related bands was performed with Δ21 antibody and the polyclonal antibody N20 raised against a peptide mapping at the amino terminus of Bax α (Figures 14B and 14C). N20 does not allow the detection of p18 band (Figure 14C), suggesting that p22 Bax is cleaved at its N-terminus moiety into a 18 kDa form. It should be noted that this early cleavage (Figure 14B) occurs with similar kinetics than caspase-2 activity (Figure 9C). Strikingly, caspase-2 inhibition or its siRNA-based genetic extinction fully abolishes Bax cleavage whereas siRNA C2 m has no effect (Figure 14D), demonstrating that caspase-2 activation is required for Bax cleavage following SD.

Cell fractionation was then performed to identify whether Bax-induced cell death during SD is mainly linked to caspase-2 activation and to check if Bax integrates into mitochondrial membrane to promote ΔΨₘ drop and cytochrome c release in cortical neurons. Bax content was analysed by Western blotting in both soluble cytosolic and mitochondria-enriched heavy membrane fractions obtained from cortical neurons subjected to 24 hr-SD with or without Z-VDVAD-FMK or siRNA C2 wt. Native p22 Bax is found in both soluble and mitochondria-enriched fractions at 24 hr-SD whereas p18 Bax is exclusively detected into the mitochondria-enriched fraction (Figure 14E). Native Bax also inserted to a lesser extent into (outer) mitochondrial membrane (Figure 14E). Said data show that both forms of Bax may participate to cell death evoked by SD. Iinvestigations were then carried out to determine whether caspase-2 dependent Bax cleavage may occur in cortical neurons in response to other stimuli. Effectively, Bax cleavage also occurs during treatment by staurosporine or ionomycin, but in these situations p18 Bax is generated in a caspase-2 independent manner (Figure 14F), confirming that other proteases may be responsible for Bax cleavage in these models (Wood *et al.,* 1998; Choi *et al.,* 2001). Accordingly, cell death induced by staurosporine or ionomycin (Figure 3D) is not prevented by Q-VD-OPH or Z-VDVAD-FMK.

The protease inhibitory profile of Bax cleavage was questioned more precisely since Bax may be cleaved directly by others cysteine proteases, calpains or through caspase-dependent calpain activation (Choi *et al.,* 2001). In order to check if calpains are responsible for Bax cleavage during SD in neurons the effect of calpains inhibitors (ALLN, ALLM and E64D) on Bax cleavage was investigated by Western-blot. In contrast to Q-VD-OPH, inhibition of calpains activity does not prevent Bax cleavage demonstrating that Bax cleavage is not directly or indirectly mediated by calpains during SD (Figure 14G). Interestingly p18 Bax appears to be stabilized by inhibition of proteasomal activity by lactacystin and epoxomycin (Figure 14H), reinforcing the previously apoptotic reported effect of p18 Bax.

All these data coincide with a model in which caspase-2 activation results in Bax cleavage into an active form.

Said results halve shown that Bax needs caspase-2 to be processed. Thus investigations were carried out to determine the biochemical status and cellular distribution of caspase-2 throughout SD. It appears that there is no up-regulation of caspase-2 mRNA following SD (Figure 14I). In contrast procaspase-2 protein content decreases in SD-neurons compared to untreated neurons and this decrease seems to be the result of a self-cleavage of caspase-2 since Z-VDVAD-FMK treatment prevents it (Figure 14J). Indeed the processed p14 form of caspase-2 is immuno-detected in SD-neurons, but not in Z-VDVAD-FMK-treated SD-neurons (Figure 14J). An intermediary product of cleavage may be also detected at 33 kDa. Kinetic analysis of caspase-2 localization during SD shows that caspase-2 is strictly cytoplasmic, even at late stage, thus ruling out a nuclear function of caspase-2 in SD cell death (Figure 14K). In contrast, several apoptogenic drugs such as the Ca²⁺ ionophore ionomycin, the kinase inhibitor staurosporine, the topoisomerase I inhibitor camptothecin, trigger partial or complete nuclear localization of caspase-2 (Figure 14L).

Thus, cytoplasmic distribution of caspase-2 in neurons is stimulus-dependent demonstrating a peculiar function of caspase-2 in the cytoplasm of SD-neurons.

### Specific caspase-2 inhibition provides strong neuroprotection during neonatal ischemic brain injury

The above results demonstrate that upstream and early caspase-2 activation are a crucial checkpoint in said *in vitro* model. Experiments were carried out to determine whether such pathway may be efficiently targeted *in vivo* during acute neuronal stress. To proceed, custom synthesis of a new cell-permeable caspase-2 inhibitor prototype was performed, named Qco-VDVAD-dfp, on the basis of the pentapeptide VDVAD combined with aminoterminal quinoline group and carboxyterminal O-phenoxy group, that may enhance both cell permeability and inhibitory potential.

SEQ ID N°5, Qco-VDVAD-dfp : Quinolinylcarbonyl-*L*-Valinyl-*L-*Aspartyl (methyl ester)-*L*-Valinyl-*L*-Alaninyl-*L*-Aspartyl (methyl ester) 2,6-difluorophenyl ester

The specificity of Qco-VDVAD-dfp was tested against recombinant caspase-2 (Figure 15A). *In vitro* VDVAD-AMC cleavage by caspase-2 is blocked by Qco-VDVAD-dfp, as efficiently as Q-VD-OPH and specific caspase-2 reversible (Ac-VDVAD-Cho) or irreversible (Z-VDVAD-FMK) inhibitors. While cleavage inhibition by Z-VAD-FMK is less important, BOC-D-FMK is completely inactive against caspase-2, thus demonstrating lower potencies of usual pan-caspase inhibitors against caspase-2. Caspase-2 is not strongly inactivated by Z-DEVD-FMK, caspase-3 like inhibitor nor by Z-LEHD-FMK, Z-LETD-FMK, caspase-3/9/8 like inhibitors respectively (Figure 15A). E64d, ALLN, ALLM inhibitors of other cysteine proteases, calpains, are unable to impair cleavage activity (Figure 16). When tested in SD paradigm (but not ionomycin-induced death,), QCO-VDVAD-DFP promotes survival of cortical neurons (Figure 15B) like Q-VD-OPH, Z-VDVAD-FMK or siRNA C2 wt did (Figures 8B, 9A and 11D and 11B), thus providing a specific caspase-2 inhibitor for *in vivo* experiments. In contrast BOC-D-FMK and Z-VAD-FMK are inefficient against SD-induced neuronal cell death (Figure 8A). We decided to test Qco-VDVAD-dfp in an acute model of hypoxic-ischemic injury in the developing brain, in which cell death occurred by apoptosis rather than. In this transient unilateral focal ischemia model, rat pups underwent permanent left middle cerebral artery occlusion in association with transient occlusion of the left common carotid artery with reperfusion .Neuroprotection effect of the pan-caspase (Q-VD-OPH) and caspase-2 specific (Qco-VDVAD-dfp) inhibitors was then examined when administrated in this perinatal ischemic model. One dose of Q-VD-OPH or Qco-VDVAD-dfp (100 µg/animal) or vehicle was administrated i.p. before the ischemic onset. Brains were then analyzed 48 hours later, a time point at which the infarct was stabilized without significant oedema (no more than 1.5 %). Ischemia induced an infarct volume of 55.0 ± 3,4 mm³, which represents a 22.1 ± 1.4 % damage in the lesioned ipsilateral hemisphere. Infarct volumes appeared normally distributed (between 15 and 26 %) (Figures 15C and 15D). A single dose of Q-VD-OPH given before ischemia, significantly reduced the infarct volume by 44 % (12.4 ± 2.6 %, p<0.05 compared to control group in the Newman-Keul's test), with volumes distributed between 0 and 31 (Figures 15D and 15E). Qco-VDVAD-dfp, at the same dose, induced a highly significant 74 % reduction in infarct volume (5.7 ± 2.3 %, p<0.01 compared to the control and Q-VD-OPH groups in the Newman-Keul's test) (Figures 15C and 15D). On the 12 studied animals, 8 displayed a very marked smaller infarct (median of 0.5%) visible at the level of the MCA occlusion (levels corresponding to plates 12 and 13) but not at that of the dorsal) and hippocampus (plate 21) compared to the ischemic control animals (Figure 15C and 15E). The four others exhibited an infarct with a mean of 16.5 ± 1.32 %, a value lower than that obtained in ischemic control animals. To conclude, our data demonstrate that specific blockade of initiator caspase-2 provides strong neuroprotection, which is more efficient than pan-caspase inhibition against ischemic brain injury.

### Discussion

### Pre-mitochondrial caspase-2 activity is required for neuronal apoptosis

The present study describes a novel intrinsic pathway subtype in which SD-induced apoptosis of primary cortical neurons is dependent on upstream activation of initiator caspase-2 that proceeds through control of Bax-induced mitochondrial dysfunction and subsequent caspase-dependent neuron destruction (Figure 17).

This model is supported by the following lines of evidence:
(i) Hierarchy and temporal orderings of apoptosis showed an intrinsic-like way in which cytosolic Bax translocates and integrates into outer mitochondrial membrane to induce ΔΨₘ drop, to promote cytochrome c release and downstream events, like cytochrome c release-dependent activation of caspases-9/caspase-3, nuclear condensation/fragmentation, PS exposure and terminal PMP.
   The results obtained according to the invention may support the formation of the classic apoptosome with cytochrome c and caspase-9. However, caspase-9 may be also involved in the activation of another downstream executioner caspases that remains to be identified since caspase-3 inhibition did not prevent terminal hallmarks of apoptosis.
(ii) Z-VDVAD-FMK promotes higher survival of neurons induced to die by SD than selective inhibitors of caspase-3, -8, -9.
(iii) Early caspase-2 activation is detected prior MMP and independently of other caspases. Pre-mitochondrial caspase-2 activation is required for SD-induced cell death since knock-down of caspase-2 by specific siRNA or pharmacological inhibition of caspase-2 activity (Z-VDVAD-FMK, Q-VD-OPH) abolishes all apoptotic hallmarks.
(iv) Inhibition of caspase-2 activity should be performed at the initiation of SD to provide cytoprotection, reinforcing the earlier and crucial role played by caspase-2.
(v) Since SD-induced apoptosis is also Bax-dependent, caspase-2 activation may mediate upstream control of Bax by allowing cleavage of native Bax into p18 fragment, independently of calpains. However both native and cleaved Bax translocate and integrate into outer mitochondrial membrane to induce ΔΨₘ drop and to promote cytochrome c release and downstream events in a caspase-2 dependent manner.
(vi)Caspase-2 is processed into a p14 form as a result of self-cleavage and remains strictly diffuse in the cytoplasm during SD, thus ruling out organelle-specific or nuclear function of caspase-2. The exclusive cytoplasmic localization of caspase-2 throughout long SD points into evidence a peculiar mechanism of activation during SD.

### Caspase-dependent versus caspase-independent neuronal cell death

Of the three broad-spectrum caspase inhibitors tested, only Q-VD-OPH, provides significant caspase inhibition and survival in cortical SD-neurons. This third generation pan-caspase inhibitor exhibits enhanced anti-apoptotic properties, not restricted to neurons, likely due to best cell permeability (aminoterminal quinoline group), specificity and effectiveness of the carboxyterminal O-phenoxy group (over classical fluoromethyl/chloromethyl ketone). Thus, Q-VD-OPH appears of greater use for neurobiology than old generation inhibitors, Z-VAD-FMK and BOC-D-FMK. Multi-caspase inhibition in neuronal culture models provided generally transient or partial protection without preservation of all apoptotic hallmarks. The reasons for this are likely due to partial mitochondrial caspase-independent pathways or activation of (upstream caspase-independent) mitochondrial pathways in which inhibition of caspase(s) involved downstream of the mitochondrial checkpoint does not prevent cytochrome c release, but rather extend the commitment to death. For example, BOC-D-FMK-saved sympathetic neurons deprived of nerve growth factor (NGF) showed a morphological preservation, without restoration of protein synthesis and of electrophysiological plasma membrane properties. Conversely, it seems that if specific caspase-2 inactivation or knock-down occurs at pre-mitochondrial level and thus prevents cytochrome c release and downstream dependent events, SD-neurons exhibit almost preserved morphology (soma and neuritic network).

As opposed to caspase activation, the role of MMP in regulation of cell death in acute and chronic neurodegenerative disorders has been reported. Nevertheless, as seen from Table I), none of the direct interference with mitochondria or PTP provides significant survival in SD-neurons. The absence of significant protection by such compounds indicates that mitochondrion is unlikely the more upstream checkpoint in SD paradigm. The data obtained according to the invention support that in some acute neuronal death models, caspase-2 acts upstream of mitochondria, and executioner caspase-3 and -9 act downstream mitochondria.

In addition, pharmacological inhibition of other signalling and metabolic major pathways failed to prevent cell death evoked by SD (see Table I). It cannot be excluded that the effect of whole compounds are bypassed and that elaborate combination may provide cytoprotection. Finally, as expected, only actinomycin D and cycloheximide promote survival of cortical neuron subjected to SD, suggesting that post-transcriptional/translational events may be involved in this death model. Indeed, *de novo* transcription and translation of macromolecules are indispensable to cell death in several neuronal apoptotic models: Cycloheximide prevented both ΔΨₘ loss and cytochrome c release in sympathetic deprived of NGF and actinomycin D blocked cell death of naive and differentiated PC12 cells deprived of NGF/serum.

### Pre-mitochondrial caspase-2 activation in SD-cortical neurons

The invention supports a model for the initial requirement of pre-mitochondrial caspase-2 that promotes high neuron survival when inactivated (Z-VDVAD-FMK) or silenced (siRNA C2wt) (Figure 8).

Strikingly caspase-2 -/- mice are viable and display no abnormal neuronal phenotype except reduction of the number of facial motor neurons (caused by accelerated apoptosis in neonatal stages and not by a decrease in neurons formation). Surprisingly, while sympathetic neurons underwent apoptosis upon NGF withdrawal and are protected by antisense caspase-2, caspase-2 deficient sympathetic neurons underwent apoptosis more efficiently than wild-type neurons. Moreover, hippocampal neurons from these mice were resistant to µ-amyloid.

Induction of transient knockdown of caspase-2 in cortical neurons by RNA interference prevents compensatory mechanisms, which allowed to demonstrate clearly the involvement of caspase-2 in neuronal death.

While subcellular localization of caspase-2 may give insight into the mechanism of its activation, its precise subcellular distribution is still controversial (Golgi complex, mitochondria, nucleus and cytoplasm), likely due to differences in cell type, death stimuli, overexpression of GFP fusion protein and antisera used to detect caspase-2. Surprisingly, caspase-2 is constitutively detected in cortical neurons as both diffuse and cytoplasmic pool, even during long SD, thus ruling out a nuclear or organelle-specific function of caspase-2 in SD cell death in cortical neurons. Both the absence of redistribution of caspase-2 in the nucleus during SD and the fact that cytoplasmic distribution of caspase-2 in cortical neurons is stimulus-dependent, suggest a peculiar mechanism of activation of caspase-2 in the cytoplasm of SD-neurons. Interestingly, seizure-induced neuronal death was also reduced by Z-VDVAD-FMK, a model in which caspase-2 was detected in both cytoplasm and nuclei of hippocampal neurons Caspase-2 staining was also mainly cytoplasmic with one to two foci in many nuclei in PC12 cells and this pattern does not change substantially in NGF-deprived cells. Altogether with SD-paradigm, these data are in favour of the role played by caspase-2 to induce apoptosis from the cytosol, which challenges the actual consensus for activation of caspase-2-mediated cell death from nuclear level.

Using sensitive ΔΨₘ dye, it was shown that *in cellula* caspase-2 activity precedes ΔΨₘ disruption and cytochrome c release in SD-neurons, which is compatible with a role played by pro-apoptotic Bcl-2 members. Said data are consistent with previous results showing that Bax is required during neuronal development and may be also critical to promote mitochondrial cytochrome c release and cell death in neurons after trophic factor deprivation

### Caspase-2 as a target during in vivo ischemia

Taking into account the difficulty to deliver siRNAs in brain, the first O-phenoxy- and quinoline-based peptide that could inhibit specifically caspase-2 was designed in order to prove the concept for *in vivo* therapeutic intervention at caspase-2 level.

Recently introduced (Melnikov *et al*., 2002; Caserta *et al*., 2003; Lecoeur *et al*., 2004), Q-VD-OPH was the only O-phenoxy- and quinoline-based inhibitor available, but was not selective. The absence of neuroprotection by Z-VAD-FMK in SD-paradigm, combined with the fact that it blocked *in vitro* caspase-2 cleavage activity, underlines the gain in cell permeability provided by the aminoterminal quinoline group. The template Qco-VDVAD-dfp used by the inventors well blocked caspase-2 activity *in vitro* and *in cellula*, thus promoting survival of SD-neurons.

SD, hypoxia or deprivation in glucose are components of in vivo cerebral or myocardial ischemia. There is evidence in neonatal models of hypoxia-ischemia (H-I) for massive apoptosis in core and penumbra rather than necrosis. Neonatal cerebral ischemia leads to delayed cell death with DNA damage and apoptotic mechanisms of cell death. Transient focal ischemia with reperfusion in the P7 rat pup leads to DNA fragmentation, morphologic features of apoptosis and activation of the mitochondrial pathway.

The inventors have demonstrated that 5 mg/kg i.p. administration of Qco-VDVAD-dfp, highly effective and cell-permeable caspase-2 inhibitor, reduces massively the infarct size (74%) in rat pups subjected to such experimental neonatal transient H-I injury. The extreme efficacy of Qco-VDVAD-dfp contrasts severely with previous results obtained in this model, showing that the pan-caspase inhibitor, BOC-D-FMK, did not induce such a significant reduction in infarct volume. Since this H-I model appears caspase-2 dependent, these findings may be consistent with our observations on the relative ineffectiveness of BOC-D-FMK in SD-neurons and against *in vitro* VDVADase activity of recombinant caspase-2. In addition this compound was not neuroprotective in spite of a previous work demonstrating significant protection following hypoxia-ischemia in the Rice-Vannucci model. In fact, BOC-D-FMK offered rather aggravation in 60 % of animals in Renolleau's model. Evidences suggest that physiological and non-lethal caspase activation contributes to axon guidance and synaptic remodelling since (i) some proteins (GluR1-4 AMPA-receptors subunits, Cam kinases, PKC interacting protein, MAP and tyrosine kinases) implied in synaptic plasticity are also substrates for caspases and (ii) Z-VAD-FMK-treated mice exhibited impaired memory. Pan-caspase inhibition in living-organism could switch from apoptosis to necrosis, tumorigenesis, or disruption of cell homeostasis, which could result in damage aggravation, cancer or auto-immune diseases. Thus alteration of physiological caspase activation, toxicity and side-effects due to prolonged administration of pan-caspase inhibitors could also limit their use in the treatment of chronic neurodegeneration, thus reinforcing the requirement for preferential selective inhibition of (initiator) caspase for both acute and chronic diseases. If partial reduction in H-I lesion could be provided by pan-caspase inhibition, whether it was due to inhibition of pro-apoptotic or pro-inflammatory caspases or both was not clear. Interestingly, since this model of neonatal stroke with reperfusion is particularly clinically relevant of neonatal human hypoxic-ischemic encephalopathy at birth, caspase-2 inhibition by small peptidic inhibitors may offer some therapeutic alternative for preservation of neurons in neonatal stroke without side-effects that may occur during pan-caspase inhibition. In addition, as specific inhibition of pro-inflammatory caspase-1-mediated processing of IL-1βand Poly(ADP-ribose) synthase (PARS) decreased also moderately cell death after ischemic injury, this may provide a rational for combining caspase-1 or PARS inhibitors with caspase-2 inhibition.

In view of the results obtained by the inventors, selective interference with pre-mitochondrial caspase-2 appears to be a relevant tool to attenuate neuronal cell death. These results allow to reconcile intrinsic pathway with orphan caspase-2 activation, at least in neuronal cell death paradigms, and to delineate a new connexion between initiator caspase and intrinsic mitochondrial pathway. Acute neuronal apoptosis may be dependent on upstream activation of initiator caspase-2 that proceeds through control of Bax-induced mitochondrial dysfunction and subsequent caspase-dependent neuron destruction. It was demonstrated that caspase-2 is also a relevant target with good neuroprotective prognosis in neonatal stroke, since *in vivo* inactivation of caspase-2 results in massive reduction of infarct volume duping transient focal ischemia.

### Experimental procedures

### Isolation and culture of primary cortical neurons

Primary cortical neurons were cultured from E14 SWISS mice embryos (Janvier). Mice were sacrificed by cervical dislocation and embryos were removed by caesarean. Cerebral cortices were extracted and tissues mechanically triturated 15 times in L15 medium (Gibco BRL) by using 1000 µl tips (Eppendorf), then debris were removed, and the cell suspension was centrifuged at 850 rpm for 10 min. Neurons were plated for 2 days at a high density (7.10⁵ live cells per cm²) in Eagle's Basal Medium (Eurobio) supplemented with 1% glutamine, 5% horse serum (HS, Eurobio) and 2.5% fetal calf serum (FCS, Eurobio) onto 6 or 24 well-plates (Sarstedt), or 4-well-Lab-Tek® chambered coverglasses (Nalge Nunc Internationnal), previously coated with 1mg/ml polyethyl- enimine (Sigma). At DIV3, medium was changed daily and neurons were maintained in N5 complete medium containing 180 mg/l glucose, 5% HS and 1% FCS, and 3 µM cytosine β-D-arabinofuranoside (Sigma) and 1 µM 5-methyl-10,11-dihydro-5H-dibenzocyclohepten-5,10-imine maleate (MK-801, Sigma). Purity of culture (> 95%) was controlled with an anti-Microtubule Associated Protein 2 monoclonal antibody (MAP-2, Sigma) and anti-Glial Fibrillary Acidic Protein polyclonal antibody (GFAP, Dako). Neurons were used between DIV6-DIV9.

### Apoptosis induction and neuroprotection assay by pharmacological agents

Cell death was induced at DIV6 by serum-deprivation (SD). Briefly, serum withdrawal was performed as followed: Neurons cultured in N5 complete medium were rapidly washed 3 times in N5 devoid of both HS and FCS, and incubated for 24 hrs in N5 medium without serum, in absence or presence of pharmacological agents. Alternatively, cell death was also induced by treatment for 24-48 hrs with ionomycin, staurosporine, camptothecin, 1-methyl-4-phenyl-1,2,3,6-tetrahydropyridine (MPTP), 3-nitropropionic acid (3NPA), sodium nitroprusside (SNP) (all purchased from Sigma) or β-amyloid peptide (25-35) (Bachem). Reagents for neuroprotection assays were added at the initiation of SD or drug treatment (in N5 complete medium). They were used at concentrations that induce no cytotoxic effect by themselves. Cyclosporin A, 4,4'-Diisothiocyanastilbene-2,2'-disulfonic acid disodium salt (DIDS), ruthenium red, decylubiquinone, acetoxymethyl ester of 1, 2-bis(2-aminophenoxy)ethane-N,N,N',N'-tetraacetic acid (BAPTA-AM), 3-methyladenine, bafilomycin A1, rapamycin, leptomycin B, N-benzyloxycarbonyl-Phe-Phe-fluoromethylketone (Z-FF-FMK), pepstatin, okadalc acid, microcystin LR, H-7, aspirin, wortmannin, genistein, lactacystin, epoxomycin, Trolox®, N-acetyl-cystein, glutathione, actinomycin D, cycloheximide were purchased from Sigma; N-benzyloxycarbonyl-Val-Ala-Asp(Ome)-fluoromethylketone (Z-VAD-FMK), BOC-Asp(OMe)-fluoromethylketone (BOC-D-FMK), Quinoline-Val-Asp(OMe)-CH₂-O-Ph (Q-VD-OPH), N-benzyloxycarbonyl-Phe-Ala-fluoromethylketone (Z-FA-FMK), N-benzyloxycarbonyl-Asp-Glu(Ome)-His-Asp(Ome)-fluoromethyl- ketone (Z-DEVD-FMK), N-benzyloxycarbonyl-Leu-Glu(Ome)-His-Asp(OMe)-fluoromethylketone (Z-LEHD-FMK), N-benzyloxycarbonyl-Leu-Glu(Ome)-Thr-Asp(OMe)-fluoromethylketone (Z-LETD-FMK), N-benzyloxy-carbonyl-Val-Asp(Ome)-Val-Ala-Asp(OMe)-fluoromethylketone (Z-VDVAD-FMK) were from ICN; custom synthesis of Quinoline-Val-Asp(Ome)-Val-Ala-Asp(OMe)-CH₂-O-Ph (Q-VDVAD-OPH) was performed by ICN; 4-(2-Aminoethyl)-benzenesulfonyl fluoride (AEBSF or Pefabloc SC) was from Roche; N-Acetyl-Leu-Leu-Norleu-al (Calpain inhibitor I or ALLN), N-Acetyl-Leu-Leu-Met-al (Calpain inhibitor II or ALLM), trans-Epoxysuccinyl-L-leucylamido-(4-guanidine)butane (E64d), MDL-28170, SB 202190, PD 98059, SP 600125 were from Merck/VWR.

### Instrumentation for dynamic analysis of apoptosis in primary cortical neurons

Multiprobe fluorescence microscopy (FM) was performed on previously stained neurons using a DM IRB inverted fluorescence microscope (Leica) equipped with a 100 W mercury short arc lamp and a X 40 N PLAN L objective or a water immersion X 100 N PLAN objective. Usually, quantitative studies were performed by both FM on approximatively 200-600 cells/field by scoring 5-10 random-selected fields per experiment and flow cytometry (FC) for higher sample throughput. For this latter, multiparametric analysis of apoptosis and related events was performed after trypsinization of stained neurons as previously described (Lecoeur *et al*., 2004). FC was performed using a 3-color FACSCalibur cytometer equipped with a 15 mW air-cooled 488 nm argon laser (Becton Dickinson).

### Multiprobe analysis of ΔΨₘ, caspase activation, PS exposure, PMP and NA.

Measurements were performed by both FC and FM, as previously described (Lecoeur *et al.,* 2004). Mitochondrial transmembrane potential (ΔΨₘ) was assessed by the ΔΨₘ-sensitive dye 5,5',6,6'-tetrachloro-1,1,3,3'-tetraethylbenzimidazolyl carbocyanine iodide (JC-1, Molecular Probes) incorporation. (Smiley et al., 1991). Neurons were loaded with 1 µM JC-1 for 30 min at 37°C. For FM, green (monomers, low Δψₘ and orange (J-aggregates, high Δψₘ) fluorescences were simultaneously acquired (BP 450-490 excitation / LP 515 long-pass emission filters). JC-1 monomers were detected in the F1-1 channel by FC. J-aggregates were detected through the F1-2 channel (Lecoeur *et al.,* 2004). Alternatively the Δψₘ was also evaluated with 60 nM MitoTracker® Red (CMXRos; Molecular Probes) and detected by FM (BP 515-560 excitation filter / LP 590 emission filter). Positive control for Δψₘ collapse was performed with carbonylcyanide *m*-chlorophenylhydrazone (mClCCP, 100 µM, 45 min). Activated caspase-2, -3, -8 and -9 were detected using specific FAM-conjugated peptides (called Fluorochrome Labeled Inhibitor of Caspase, FLICA: CaspaTag™ fluorescein Caspase Activity Kits, Q-Biogen, Illkirch, France; ApoFluor™ Caspase Detection Kits, ICN, Orsay, France): FAM-VDVAD-FMK, FAM-DEVD-FMK, FAM-LETD-FMK and FAM-LEHD-FMK, respectively. Neurons were incubated with FLICAs (1:150, CaspaTag™ or 1:500, ApoFluor™) for 1 hr at 37°C, then washed three times in washing buffer. For FM, FAM-conjugated peptides were excited through the BP 480/40 filter and the emitted light was collected through the BP 527/30 filter. FC analysis was performed in F1-1 channel (Lecoeur *et al.,* 2004). Phosphatidylserine (PS) exposure to the outer leaflet of the plasma membrane was detected through the fixation of FITC conjugated- annexin V (Immunotech). The plasma membrane permeability (PMP) was detected through increased binding of 7-Amino Actinomycin D (7-AAD, Sigma) to nuclear DNA. Stainings and analysis by FM and FC were' performed as previously (Lecoeur *et al.,* 2004). Nuclei were stained with 1 µM Hoechst 33342 (30min) and analyzed by FM (BP 340-380 excitation filter/LP 425 long-pass filter). Nuclear apoptosis (NA) was evaluated as previously defined in neurons (Lecoeur *et al.,* 2004).

### Immunodection of cytochrome c, Bax, caspase-2 and caspase-3

Neurons grown in Lab-Tek® chamber slides were fixed in 4% paraformaldehyde/0.19% picric acid for 20 min, permeabilized with 0.01% Triton-X100 in PBS for 5 min, then blocked with 10% FCS in PBS for 30-45 min. All immunostainings were performed at RT. Antibodies were diluted in 1% bovine serum albumin (Sigma) in PBS. Then, neurons were stained using the mouse monoclonal IgG1 anti-cytochrome c (1 hr, 1:200; clone 6H2 . B4, BD Pharmingen) and a Alexa Fluor® 594 F (ab')₂ fragment of goat anti-mouse IgG (1 hr, 1:200; Molecular Probes), as secondary antibody. Similarly, Bax translocation was investigated using a rabbit polyclonal antibody raised against mouse Bax α deleted for the carboxy terminal 21 amino acids (1h, 1:100; Δ21, Santa Cruz Biotechnology) and detected with a FITC-goat anti-rabbit IgG antibody (1h, 1:100; Molecular Probes). Cells displaying either a diffuse cytoplasmic cytochrome c or a Bax punctuate labelling were counted under FM on about 10 fields corresponding to 150-300 randomly chosen cells per condition per experience. Caspase-2 was detected *in cellula* by using the rat monoclonal anti-mouse caspase-2 antibody (10C6, Alexis Biochemicals, San Diego, CA, USA; 1:100, 1h) and an Alexa Fluoro® 594 F(ab')₂ fragment of goat anti-rat IgG (1 hr, 1:100, Molecular Probes) as secondary antibody. Activated caspase-3 was evidenced *in cellula* by FC (Lecoeur *et al.,* 2004). To proceed, neurons were trypsinized, fixed in PBS containing 1% PFA and 20 µg/ml actinomycin D (Sigma) for 20 min. Then, neurons were resuspended in 100 µL PBS/1% BSA/0.05% saponin Quilaja bark (Sigma) containing both 20 µg/ml 7-AAD and 20 µl of the Phycoerythrin-conjugated polyclonal rabbit anti-caspase-3 antibody (BD Pharmingen,) for 30 min.

### RNA Interference

Double stranded siRNA corresponded to the sequence of the mouse *Caspase-2* gene (AACACCTCCTAG AGAAGGACA; nucleotides 185-203; siRNA C2 wt). Inactive siRNA was designed with four mutations in the same sequence (AACATCTACTCG AGACGGACA; siRNA C2 m). siRNA C2 wt sequence was submitted to BLAST to ensure its specificity. Annealed siRNAs duplexes (RP-HPLC purified) were purchased from Proligo. Neuronal cultures at DIV6 in 24 well-plates (7.10⁶/well) or Lab-Tek ® 4-chambered cover glasses (1.33.10⁶/well) were transfected for 6h with siRNAs (3.8 µg) using Lipofectamine 2000 (Invitrogen). Then neurons were washed and returned to complete N5 medium for further 16 hrs, prior to be subjected,'or not, to 24 hr-SD or ionomycin treatment.

### RT-PCR analysis

RNA extraction was performed directly in 24-well (1.33x10⁶ neurons) or 6-well (7x10⁶ neurons) plates with the RNeasy mini Kit (Qiagen) according to manufacturer's recommendations. The reverse transcription was performed using Supercript^{™} II RNase H⁻ reverse transcriptase (Invitrogen). PCR primers were purchased from Proligo: *Bax* forward primer 5'-AGAGGCAGCGGCAGTGAT-3', *Bax* reverse primer 5'- AGACACAGTCCAA GGCAGTGG-3';caspase-2 forward primer 5'-GAGCAATGTGCACTTCACTGG-*3', caspase-2* reverse primer 5'- CCACACCATGTGAGAGGAGTG-3'; *caspase-9* forward primer 5'-AGCTGGAGCCGTCACAGCC-3', *caspase-9* reverse 5'-CTCCGCCAGAACCAATGTCC-3'; *GAPDH* forward primer 5'-GGTCGGAGTCAACGG ATTTGGTCG-3', *GAPDH* reverse primer 5'-CCTCCGACGCCTGCTTCACCAC-3'. The amplification conditions were 94°C for 1 min, followed by: 30 cycles for *Bax* at 94°C for 30 s, 58°C for 30 s, 72°C for 1 min then 72°C for 15 min; 35 cycles for *caspase* -2 and *caspase-9* or 25 cycles for *GAPDH* at 94°C for 30 s, 54°C for 30 s, 72°C for 1 min then 72°C for 15 min. After PCR, 20 µl were subjected to electrophoresis on 1.5% agarose gels and bands were visualized by UV. transillumination with ethidium bromide staining prior to photography. *GAPDH* is used as an internal control of amplification.

### Cytosol preparation and subcellular fractionation

Neurons (7x10⁶ in 6-well plate) were harvested at 4°C in 50 µl of CSF buffer (220 mM mannitol, 68 mM sucrose, 5 mM pyruvate, 0.5 mM EGTA, MgCl₂ 2 mM, NaCl 2 mM, KH₂PO₄ 2.5 mM, dithiothreitol 1 mM, cytochalasine B 20 µM and 10 mM Hepes, pH 7.5) supplemented with complete protease inhibitors cocktail (Roche), then broken five freeze-thaw cycles in liquid nitrogen. Samples were centrifuged at 900g for 5 min at 4°C to remove nuclei and unbroken cells, followed by centrifugation at 10,000 g for 30 min at 4°C to obtain the heavy membrane fraction enriched in mitochondria. Then samples were centifuged at 100,000 g for 10 min at 4°C to pellet microsomes. Material was resuspended in 25 mM Tris-HCl pH 7.4, 25 mM NaCl, 5 mM EDTA, 1% Triton X-100 prior to protein concentration determination by Bradford assay method. 10 µg of each fraction was used for Western blot analysis.

### Protein extraction and Western Blot analysis

Neurons were lysed at RT in 25 mM Tris-HCl pH 7.4, 25 mM NaCl, 5 mM EDTA, 1% Triton X-100 supplemented by complete protease inhibitors cocktail (Roche). Protein concentration was determined using the Bio-Rad protein assay kit. Proteins (30 µg for caspase-2; 10 µg for Bax) were separated on 12.5% polyacrylamide gels and transferred to PVDF membranes (Amersham). Immunostaining was revealed using ECL (Amersham Pharmacia Biotech). The monoclonal anti-mouse caspase-2 antibody (11B4, Alexis Biochemicals) was used at a 1:1000 dilution; polyclonal antibody (Δ21, Santa Cruz Biotechnology) raised against mouse Bax α deleted for the carboxy terminal 21 amino acids was used at a 1:200 dilution; polyclonal antibody (N20, Santa Cruz Biotechnology) raised against the amino terminus of Bax α (recognizing residues 11 to 30) was used at a 1:1000 dilution. Actin (42kDa; Sigma; 1:5000) is used as an equal loading control. Immunoblotting of heat-shock protein 60 (HSP60) with a mouse monoclonal anti-HSP (Sigma; 1:400) was used to check the purity of the heavy membrane fraction enriched in mitochondria.

### In vitro VDVAD-AMC cleavage by recombinant caspase-2

Activity of human recombinant caspase-2 (BIOMOL QuantiZyme™ Assay System) was assessed in 100µl assay buffer (50 mM HEPES, pH 7.4, 100mM NaCl, 0.1% CHAPS, 10 mM DTT, 1mM EDTA, 10% glycerol). The cleavage of 50 µM VDVAD-AMC by recombinant caspase-2 (125 U) was measured after 30 min at 37°C on a fluorescence microplate reader by monitoring the fluorescence emission at 510 nm upon excitation at 405 nm. For inhibition of VDVADase activity, inhibitors (2 µM) were pre-incubated 30 min at 37°C in presence of caspase-2 prior to subsequent incubation with 50 µM VDVAD-AMC (30 min, 37°C). No noticeable fluorescence background was observed with VDVAD-AMC alone.

### Perinatal ischemia

Newborn Wistar rats (dam plus 9 pups per litter) were obtained from Janvier (Le Genest-St-Isle, France) when the pups were 3-4 days of age. The pups were housed with their dam under a 12:12 h light-dark cycle with food and water freely available. Animal experimentation was conducted according to the French and European Community guidelines for the care and use of experimental animals. Ischemia was performed in 7 day-old rats (17-21 g), as previously described (Renolleau *et al.*, 1998). Rat pups were anesthetized with an intraperitoneal injection of chloral hydrate (350 mg/kg). Anesthetized rats were positioned on their back and a median incision was made in the neck to expose the left common carotid artery. Rats were then placed on the right side and an oblique skin incision was made between the ear and the eye. After excision of the temporal muscle, the cranial bone was removed from the frontal suture to a level below the zygomatic arch. Then, the left middle cerebral artery, exposed just after its appearance over the rhinal fissure, was coagulated at the inferior level of the cerebral vein. After this procedure, a clip was placed to occlude the left common carotid artery. Rats were then placed in an incubator to avoid hypothermia. After 50 min, the clip was removed. Carotid blood flow restoration was verified with the aid of a microscope. Neck and cranial skin incisions were then closed. During the surgical procedure, body temperature was maintained at 37-38°C. Pups were transferred in an incubator (32°C) until recovery then after to their dams.

Caspase inhibitors were administered intraperitoneally at a dose of 50 µg per 10g weight (in 100 µl) 5 min before the ischemic onset (n= 15 for Q-VD-OPH, n= 14 for Qco-VDVAD-dfp). Control animals received an equivalent volume of 0.9 % saline containing 10% DMSO (n=15), the vehicle required to solubilize the caspase inhibitors (vehicle-treated group). The mortality rate during ischemia or before killing did not differ between Q-VD-OPH-, Qco-VDVAD-dfp - and vehicle-treated groups (< 4%). Rats were killed 48 hours after reperfusion and brains were removed. The infarct lesion (pale zone) was visually scored by an observer blinded to the treatment of animals. Brains without a clear ischemic pale zone were observed under a magnifying glass. Those exhibiting no clear MCA occlusion were discarded (2 animals in the Q-VD-VAD-treated group). Brains were then fixed 2 days in 4% buffered formaldehyde. Fifty-micrometer coronal brain sections were cut on a cryostat and collected on gelatin-coated slides. Sixteen sections from anterior striatum to posterior hippocampus (corresponding to plates 9 to 27 in Paxinos' rat brain atlas) were selected, taken at equally spaced 0.5-mm intervals. The lesion areas were measured on cresyl violet-stained sections using an image analyzer (NIH image software), and the distances between respective coronal sections were used to calculate the infarct volume.

Statistical analysis was performed as followed. Assuming a beta risk of 0.2 and an alpha risk of 0.05, it was estimated that 15-16 animals in each group were needed to detect a 50% infarct volume reduction between two groups. Data were drawn from previous study (Ducroq et al., 2000). Because three groups of animals are compared in the experiments, these values are only informative. A predetermined list with blocks of six animals was used to randomized the animals among the three groups. An investigator blind to the treatment condition did all measurements. The difference between the means was assessed by the non-parametric multiple comparison test of Kruskall-Wallis, followed by the Newman-Keul's test for non-parametric values. We consider differences to be significant at the 5 % level (*P*<0.05).

### Ref. EXAMPLE IV: Design of a specific siRNA for human caspase-2 silencing

Specific siRNA (hsiRNAC2 wt) for human caspase-2 gene knock-down was designed for further applications to human (ischemic and others) injuries and diseases. This siRNAs duplex is composed of the following complementary sequences:
SEQ ID N°6 5'-caucuucuggagaaggacadTdT-3'
SEQ ID N°7' 5'-uguccuucuccagaagaugdTdT-3'

An experimental approach was developed to test said siRNA based on the model of Robertson (Robertson et al., 2002), that showed that caspase-2 inhibition by Z-VDVAD-FMK decreased partially cytochrome c release and phosphatidylserine residues exposure in Jurkat T cells.

Pharmacological caspase-2 inhibition (Z-VDVAD-FMK, Q-VD-OPH; all from ICN) or caspase-2 gene knockdown (siRNA) in VP-16 treated-Jurkat cells were then performed.

### siRNA validation in human cells

Pre-treatment by the pan-caspase Q-VD-OPH (25-100µM) or the selective caspase-2 inhibitor, Z-VDVAD-FMK (25-100µM) prevents cell death induced by the DNA-damaging and topoisomerase II ihibitor, VP16 (Figure 18). The survival at 7-8 hrs was obtained against a large range of concentration of VP16 (Figure 18). The fact that Z-VDVAD-FMK blocked Δψₘ loss suggest that caspase-2 activation occurs upstream of mitochondria in this paradigm. Accordingly in Figure 19, data show that:
(i) the progressive Δψₘ loss is not abolished by Z- DEVD -FMK, Z-LEHD-FMK, Z-LETD-FMK, but only by Z-VDVAD-FMK or Q-VD-OPH;
(ii) Z- DEVD -FMK, Z-LEHD-FMK, Z-LETD-FMK do not impair caspase-2 activation suggesting that caspase-2 is the more upstream caspase studied;
(iii) caspase-9 inhibition prevent caspase-3 activation but caspase-3 inhibition does prevent caspase-9 activation, showing that caspase-3 is activated through caspase-9;
(iv) terminal nuclear alterations and PMP are moslty prevent by Z-VDVAD-FMK, Q-VD-OPH and to a lesser extent by Z-LEHD-FMK;
(v) the ANT-blocker BA, attenuates Δψₘ, loss and PMP confirming the role of mitochondria in mediating the pro-apoptotic effect of activated caspase-2;
(vi) VP16-caspase-2 dependent cell death is not dependent on translation and transcription, since CHX and ActD prevent neither Δψₘ loss nor PMP;
(vii) Caspase-8 dependent pathway is not important in this model because Z-LETD-FMK is unable to prevent Δψₘ loss, caspase-2 and -3 activation, nuclear alteration and PMP. Finally, the whole data point into evidence a model in which pre-mitochondrial caspase-2 activation induce Δψₘ drop, and promotes downstream events, like activation of caspases-9/caspase-3 activation, nuclear condensation/fragmentation and terminal PMP.

This paradigm has allowed testing and validating of human siRNA directed to caspase-2. First, hsiRNA C2 wt is able to decrease pro-caspase-2 protein expression in HeLa and Jurkat cells, respectively (as shown by Western Blot analysis in Figure 20 A). All cells are tranfected as assessed by *in cellula* by fluorescence detection of siRNA-FITC by flow cytometry. Once these cells are transfected, they are also protected against subsequent 7hr-treatment with VP16 (Figure 21A-B), demonstrating the validity of the hsiRNA C2 wt.

### Experimental section

### Cell culture:

Jurkat cells were purchased from ATCC (clone E6-1) and were cultured at density of 100000-120000 cells/well (24-wells plate) in RMPI 1640 (Glutamax rich) medium supplemented with 10% foetal bovine serum. Jurkat E6-1 cell (ATCC number: TIB-152) is a clone of the Jurkat-FHCRC, a derivative of the Jurkat cell line (previously established from peripheral blood of a 14 year old boy by Schneider et al. (1977) and that was originally designed JM). Cells were used at passages 7-14 for experiments.

### Apoptosis induction and cytoprotection assay

Cells were pretreated with various pharmacological agents for 30min-1hr, prior to subsequent VP16 (VP16 or etoposide; Sigma) treatment (10-20 µM) for 7-8 hrs. For siRNA experiments, cells were treated for 24 hrs with 3.8 µg siRNA (Proligo)/2 µL lipofectamine 2000 (in 500 µL), before VP16 treatment. Murine caspase-2 (ID N°1-2 or ID N°3-4) was used for negative control. Transfection yield was checked *in cellula* by fluorescence detection (flow cytometry, FL-1) of siRNA-FITC (ID N°1-2 , ID N°3-4 or ID N°6-7)

### Apoptosis parameters study by flow cytometry and fluorescence microscopy

### Flow cytometry

Double JC-1/7AAD staining: Mitochondrial transmembrane potential (Δψₘ) was assessed by the DYₘ-sensitive dye 5,5',6,6'-tetracholoro-1,1,3,3'-tetraethylbenzimidazolyl carbocyanine iodide (JC-1, Molecular Probes, 1µm) incorporation. Green (low (Δψₘ) and orange (high Δψₘ) fluorescences were respectively acquired in F_{L}-1 and FL-2 channels, respectively. PMP was detected by 7-actinomycin D (7AAD; 0.02 mg; Sigma) incorporation (FL-3 channels). Alternatively, double DioC6 (0.1 µM)/PI (5.10⁻³ mg) staining was performed and detected in FL-1 and FL-2 channels, respectively. 7000 events are at least acquired for each condition.

### Fluorescence microscopy

Activated caspase-2, -3, and -9 were detected using specific FAM-conjugated peptides (called Fluorochrome Labeled Inhibitor of Caspase, FLICA: CaspaTag^{™} fluorescein Caspase Activity Kits, Q-Biogen, Illkirch, France; ApoFluor^{™} Caspase Detection Kits, ICN, Orsay, France): FAM-VDVAD-FMK, FAM-DEVD-FMK, FAM-LETD-FMK and FAM-LEHD-FMK, respectively. Cells were incubated with FLICAs (1:150, CaspaTag ^{™} or 1:500, ApoFluor ^{™}) for 1 hr at 37°C, then washed three times in washing buffer. For FM, FAM-conjugated peptides were excited through the BP 480/40 filter and the emitted light was collected through the BP 527/30 filter. The plasma membrane permeability (PMP) was detected through increased binding of 7-Amino Actinomycin D (0.02 mg 7-AAD, Sigma) to nuclear DNA (excited through the BP515-560 filter and fluorescence collected through the LP590 long pass emission filter). Nuclei were stained with 1 µM Hoechst 33342 (30min) and analyzed (BP 340-380 excitation filter/ LP 425 long-pass filter). Mitochondrial transmembrane potential was assessed by JC-1 (1 µM, 30 min): green and orange fluorescences were simultaneously recorded after 1.2 s excitation (BP 450-490 excitation / LP 515 long-pass emission filters).

### Ref. EXAMPLE V: shRNA

### shRNA construction and validation

Even if siRNA are able to cross the blood brain barrier, they are unstable in biological fluids, thus the difficult obstacle to overcome will be in vivo intracellular delivery. Recently, several breakthrough have highlighted viruses as excellent vehicles for siRNA delivery. For example, retroviruses or adenoviruses, the transgene-delivery vectors of choice for many experimental gene therapy studies, have been engineered to deliver and stably express therapeutic siRNA within cells, both in vitro and in vivo. Indeed, recombinant versions of siRNA: small hairpin (sh)RNA (constitutive siRNA expression as hairpin loop version under control of a small RNA promoter) have been produced to circumvent this problem. ShRNA expression can be induced in lentiviral backbone for example, that could be used to stably transfect neurons in vivo, by local brain admistration (intracerebro-ventricular injection for example), which should lead to the permanent silencing of the target gene.

SIn order to generate in cellula stable siRNA structure, the concept of small hairpin structure have been developed consisting on the expression of the sens and antisens sequences of the siRNA linked by a short sequence and followed by the termination signal (TTTTT) of the pol III polymerase. This sequence is under the control of pol III promoters from either the H1 RnaseP or U6 small nuclear RNA genes and lead to the expression of large amount of small hairpin siRNA (shRNA) in transfected cells. A rapid processing of the loop part certainly by DICER leads to the formation of functional siRNA. Recently a plasmid (pGE-1) has been developed (Stratagene) and we used this shRNA mammalian expression vector to provide efficient long-term suppression of the target gene. The shRNA is generated from an RNA transcript (controlled by a U6 promoter) that consists of sens and antisens strands separated by a loop sequence. The RNA transcript folds back on itself to form a hairpin. The pGE-1 expression vector has been optimized for suppressing expression of target genes in mammalian cells. In order to obtain an expression vector containing the shRNA specific for murine caspase-2 two oligonucleotides were designed (Figure 22A), consisting of two inverted repeats separated by a loop sequence and followed by a 6 nucleotide poly(T) string which serves as a transcription terminator for the RNA polymerase III.
SEQ ID N°8 5'-GATCCCgcacctcctagagaaggacaGAAGCTTGtgtccttctctaggaggtgTTTTTT-3'
SEQ ID N°9 5'-CTAGAAAAAAcacctcctagagaaggacaCAAGCTTCtgtccttctctaggaggtgCGG-3'

Following the annealing of the two oligonucleotides we obtained a sh-insert (Figure 22B) which was cloned into the BamH I and Xba I sites of the pGE-1 vector. After screening of positive colonies by PCR we selected 2 clones (shRNA6 and shRNA9). These clones were sequenced and showed the right insertion of the sh-sequence under the control of the U6 promoter.

In order to validate these shRNA constructs as a tool for caspase-2 down regulation, 3T3 cells (murine cells) were transfected with the vectors shRNA6 and shRNA9 and checked the level of expression by Western Blot of caspase-2 in total extracts of the 3T3 cells 24 and 48 hours post-transfection (Fig.23).

It appears that both shRNA6 and shRNA9 constructs are able to down regulate the expression of caspase-2 in 3T3 cells 48 hours after transfection. This result shows that a shRNA strategy is useful as a tool for *in vivo* silencing of caspase-2 expression. Indeed the sh-insert targeting caspase-2 mRNA could be introduced in several viral backbones (lentivirus, adenovirus, Semliki virus or any viral backbone with a therapeutic field of application) thus permitting an efficient *in vivo* delivery and an efficient and long-term silencing of caspase-2 expression.

In addition, specific shRNA construct has been obtained for application to humans :
SEQ ID N°10 5'-GATCCCGcatcttctggagaaggacaGAAGCTTGtgtccttctccagaagatgTTTTTT-3'
SEQ ID N°11 5'-CTAGAAAAAAcatcttctggagaaggacaCAAGCTTCtgtccttctccagaagatgCGG-3'

### Experimental section

Two complementary oligonucleotides with 5' BamH I and 3' Xba I overhangs has been synthesized (Proligo). After an annealing step, these oligonucleotides were cloned into a predigested (BamH I/Xba I) pGE-1 vector (Stratagene). Following PCR selection of positives clones containing the insert, two clones were amplified and their sequence verified (shRNA6 and shRNA9).

3T3 cells plated in 6 wells dishes the day before were transfected using lipofectamine 2000 reagent and 0.8 µg of shRNA6 or shRNA9 plasmids during 6 hours. Level of tranfection was monitored using a GFP vector. 24 and 48 hours after the transfection, cells were harvested in lysis buffer (25 mM Tris-Hcl pH 7.4, 25 mM NaCl, 5 mM EDTA, 1% Triton X-100) and protein concentration was determined using the Bradford Reagent (BioRad). Proteins (20 µg per sample) were separated on 12.5% polyacrylamide gels (SDS-PAGE) and transferred on PVDF membranes (Amersham). After probing with an anti-mouse monoclonal antibody specific for caspase-2 (11B4, Alexis Biochemicals, used at a 1:1000 dilution), immunoreactivity was detected with a chemiluminescence kit (ECL, Amersham).

**Table 1. Caspases play a crucial role in the regulation of SD-related apoptosis of primary cortical neurons.**

| Compounds tested | [], µM | Specific targets/Activity | Survival |
|---|---|---|---|
| Actinamycin D | 0.016 | RNA synthesis | Yes |
| Cycloheximide | 1 | de novo protein synthesis | Yes |
| Q-YD-OPH | 100 | Broad-spectrum caspases | Yes |
| z-VAD-fmk | 100 | Broad-spectrum caspases | No |
| BOC-D-fmk | 100 | Broad-spectrum caspases | No |
| Decyl-Ubiquinone | 10 | Complex III repiratory chain; PTP | No |
| DIDS | 50 | Anionic channels; VDAC (PTP) | No |
| Cyclosporin A | 1 | Cyclophyrin D (PTP) | No |
| Ruthenium Red | 50 | Mitochondrial calcium uptake; VDAC (PTP) | No |
| Rapamycin | 1 | Mammalian Target of Rapamycin (mTOR) or | No |
| | | FKBP12-Rapamycin-Associated Protein (FRAP) | No |
| 3-Methyledenine | 1000 | Lysosomal pH (alkalinization induction) | No |
| Bafilomycin A1 | 1 | Lysosomal vacuolar type H*-ATPase | No |
| z-FA-fmk | 100 | Cathepsin B-like activity | No |
| z-FF-fmk | 150 | Cathepsin L-like activity | No |
| Pepstatin | 50 | Carthepsin D-like activity | No |
| E64d | 100 | Calpains + cathepsins B,H,L-like activities | No |
| ALLN | 25/150 | Calpain I/20 S Proteasome | No/No |
| ALLM | 25/100 | Calpain II/ 20 S Proteasome | No/No |
| MDL-28170 | 1-100 | Calpain I + I] | No |
| Pefabloc AEBSF | 100 | Serine protease activity | No |
| Lactacystin | 0.1-10 | 20S Proteasame | No |
| Epoxomicin | 0.1-10 | 20S Proteasome | No |
| BAPTA-AM | 50 | Selective chelation of cytosolic calcium stores | No |
| Aminopurvalanol | 500 | Cyclin-Dependent Kinases (CDX) 1,2,5 | No |
| Roscovitine | 250 | CDK1,2,5 | No |
| SB 202190 | 50 | p38 Mitogen-Activated Protein Kinase (MAPK) | No |
| PD 38059 | 50 | Mitogen-Activated Protein Kinase Kinase (MEKI) | No |
| SP 600125 | 50 | Jun N-terminal Kinases (JNK) | No |
| Genistein | 100 | Tyrosine Kinases | No |
| Wonnannin | 100 | Phosphoinositide 3' (PI₃) Kinase | No |
| Aspirin | 100 | IKK | No |
| H-7 | 100 | PKC (>> PKA(PKG) | No |
| Okadaïc acid | 0.01 | Phosphatase : PP2A | No |
| Microcystin LR | 1-100 | Phosphatases : PP1 + PP2A | No |
| Trolox⊙ | 100-1000 | Antioxidant | No |
| N-Acetyl-Cystein | 100-1000 | Antioxidant | No |
| glutathion | 100-1000 | Antioxidant | No |
| Leptomycin B | 0.05 | Nucleocytoplasmic translocation of | No |
| | | proteins containing a nuclear **export signal** | No |

The table shows wide-ranging classes of pharmacological agents tested in SD model that were able or not to promote survival, i.e, preventing Δψₘ collapse, NA, PS exposure, PMP, caspases activation and neumes alterations. All these compounds are added at the start of SD. VDAC : voltage-dependent-anionic-channel; PTP : permeability transition pore.

### REFERENCES

Caserta, T.M., Smith, A.N., Gultice, A.D., Reedy, M.A., and Brown, T.L. (2003). Q-VD-OPh, a broad spectrum caspase inhibitor with potent antiapoptotic properties. Apoptosis 8, 345-352.
Chang, L.K., and Johnson, E.M. Jr. (2002). Cyclosporin A inhibits caspase-independent death of NGF-deprived sympathetic neurons: a potential role for mitochondrial permeability transition. J. Cell Biol. 157, 771-781.
Choi, W.S., Lee, E.H., Chung, C.W., Jung, Y.K., Jin, B.K., Kim, S.U., Oh, T.H., Saido, T.C., and Oh, Y.J. (2001). Cleavage of Bax is mediated by caspase-dependent or - independent calpain activation in dopaminergic neuronal cells: protective role of Bcl-2. J. Neurochem. 77, 1531-1541.
Deckwerth, T.L., Elliott, J.L., Knudson, C.M., Johnson, E.M. Jr, Snider, W.D., and Korsmeyer, S.J. (1996). BAX is required for neuronal death after trophic factor deprivation and during development. Neuron. 17, 401-411.
Deshmukh, M., Vasilakos, J., Deckwerth, T.L., Lampe, P.A., Shivers, B.D., and Johnson, E.M. Jr. (1996). Genetic and metabolic status of NGF-deprived sympathetic neurons saved by an inhibitor of ICE family proteases. J. Cell Biol. 135, 1341-1354.
Deshmukh, M., and Johnson, E.M. Jr. (1998). Evidence of a novel event during neuronal death: development of competence-to-die in response to cytoplasmic cytochrome c. Neuron 21, 695-705.
Ethell DW and Green DR (2002) Assessing Cytochrome-c release from mitochondria.In:Apoptosis techniques and protocols. (Humana Press), pp 21-34
Johnson EJ (1995) Methods for studying cell death and viability in primary neurons. Meth Cell Biol 46 243-276.
Kawamoto JC, and Barrett JN (1986) Cryopreservation of primary neurons for tissue culture. Brain Res 384: 84-93.
Knusel B, Michel PP, Schwaber JS, and Hefti F (1990) Selective and nonselective stimulation of central cholinergic and dopaminergic development in vitro by nerve growth factor, basic fibroblast growth factor, epidermal growth factor, insulin and the insulin-like growth factors I and II. J Neurosci 10: 558-570.
Lecoeur H, de Oliveira Pinto L, and Gougeon ML (2002) Multiparametric flow cytometric analysis of biochemical and functional events associated with apoptosis and oncosis using the 7-aminoactinomycin D assay. J Immunol Methods 265: 81-96.
Lecoeur H, Fevrier M, Garcia S, Riviere Y and Gougeon ML (2001) A novel flow cytometric assay for quantitation and multiparametric characterization of cell-mediated cytotoxicity. J Immunol Methods 253: 177-187.
Lecoeur, H., Chauvier, D., Langonné, A., Rebouillat, D., Brugg, B., Mariani, J., Edelman, L., and Jacotot, E. (2004). Fixed- and real-time cytofluorometric technologies for dynamic analysis of apoptosis in primary cortical neurons. Apoptosis 9, 157-169.
Lipton, P. (1999). Ischemic cell death in brain neurons. Physiol. Rev. 79, 1431-1568.
Melnikov, V.Y., Faubel, S., Siegmund, B., Lucia, M.S., Ljubanovic, D., and Edelstein, C.L. (2002). Neutrophil-independent mechanisms of caspase-1- and IL-18-mediated ischemic acute tubular necrosis in mice. J. Clin. Invest. 110, 1083-1091.
Plesnila N, Zinkel S, Le DA, Amin-Hanjani S, Wu Y, Qiu J, Chiarugi A, Thomas SS, Kohane, DS, Korsmeyer SJ, and Moskowitz MA (2001) BID mediates neuronal cell death after oxygen/glucose deprivation and focal cerebral ischemia. Proc Natl Acad Sci U S A. 98: 15318-15323.
Robertson, J.D., Enoksson, M., Suomela, M., Zhivotovsky, B., and Orrenius, S. (2002). Caspase-2 acts upstream of mitochondria to promote cytochrome c release during etoposide-induced apoptosis. J. Biol. Chem. 277, 29803-29809.
Schneider U, Schwenk HU, Bornkamm G. Characterization of EBV-genome negative "null" and "T" cell lines derived from children with acute lymphoblastic leukemia and leukemic transformed non-Hodgkin lymphoma. Int J Cancer. 1977, 19 :621-626.
Smolewski P, Grabarek J, Halicka HD, and Darzynkiewicz Z (2002) Assay of caspase activation in situ combined with probing plasma membrane integrity to detect three distinct stages of apoptosis. J Immunol Methods 265: 111-121.
Susin, S.A., Lorenzo, H., Zamzami, N., Marzo, I., Snow, B.E., Brothers, G.M., Mangion, J., Jacotot, E., Costantini, P., Loeffler, M., Larochette, N., Goodlett, D.R., Aebersold, R., Siderovski, D.P., Penninger, J.M., and Kroemer, G. (1999a). Molecular characterization of mitochondrial apoptosis-inducing factor. Nature 397, 441-446.

## Claims

1. A compound suitable for inhibition of caspase-2 activity having SEQ ID N°5: quinolinylcarbonyl-L-Valinyl-L-Aspartyl(methyl ester)-L-Valinyl-L-Alaninyl-L-Aspartyl(methyl ester)2,6-difluorophenyl ester.

2. A pharmaceutical composition comprising a therapeutically effective amount of the compound of claim 1 in association with a pharmaceutically acceptable carrier.

3. A compound having SEQ ID N°5: quinolinylcarbonyl-L-Valinyl-L-Aspartyl(methyl ester)-L-Valinyl-L-Alaninyl-L-Aspartyl(methyl ester)2,6-difluorophenyl ester for use as a medicament.

4. A compound having SEQ ID N°5: quinolinylcarbonyl-L-Valinyl-L-Aspartyl(methyl ester)-L-Valinyl-L-Alaninyl-L-Aspartyl(methyl ester)2,6-difluorophenyl ester for use in treatment of a pathological situation including hypoxia-ischemia, H-I, injuries and stroke-like situations.

5. A compound for use according to claim 4, wherein the H-I injury is cerebral hypoxia-ischemia.

6. A compound for use according to claim 4, wherein the pathological situation is neuronal death, particularly in global or focal hypoxia-ischemia.

7. A compound for use according to claim 4, wherein the pathological situation is neuronal death, particularly in adult or neonatal hypoxia-ischemia.

8. A compound for use according to claim 4, wherein the pathological situation is neuronal death, particularly in transient or permanent hypoxia-ischemia.

9. A compound according to claim 4, wherein the pathological situation is neuronal death, particularly in Middle Cerebral Artery Occlusion (MCAO).

## Patentansprüche

1. Verbindung, die zur Inhibierung von Caspase-2-Aktivität geeignet ist, die SEQ ID NO: 5 hat: Chinolinylcarbonyl-L-valinyl-L-aspartyl(methylester)-L-valinyl-L-alanyl-L-aspartyl(methylester)2,6-difluorphenylester.

2. Pharmazeutische Zusammensetzung, die eine therapeutisch wirksame Menge der Verbindung gemäß Anspruch 1 in Verbindung mit einem pharmazeutisch annehmbaren Träger umfasst.

3. Verbindung, die SEQ ID NO: 5 hat: Chinolinylcarbonyl-L-valinyl-L-aspartyl(methylester)-L-valinyl-L-alanyl-L-aspartyl(methylester)2,6-difluorphenylester zur Verwendung als Medikament.

4. Verbindung, die SEQ ID NO: 5 hat: Chinolinylcarbonyl-L-valinyl-L-aspartyl(methylester)-L-valinyl-L-alanyl-L-aspartyl(methylester)2,6-difluorphenylester zur Verwendung bei der Behandlung eines pathologischen Zustandes, der Hypoxie-Ischämie, H-I-Verletzungen und schlaganfallartige Zustände umfasst.

5. Verbindung zur Verwendung gemäß Anspruch 4, wobei die H-I-Verletzung zerebrale Hypoxie-Ischämie ist.

6. Verbindung zur Verwendung gemäß Anspruch 4, wobei der pathologische Zustand neuronaler Tod, insbesondere bei globaler oder fokalier Hypoxie-Ischämie ist.

7. Verbindung zur Verwendung gemäß Anspruch 4, wobei der pathologische Zustand neuronaler Tod, insbesondere bei Erwachsenen- oder Neugeborenen-Hypoxie-Ischäme, ist.

8. Verbindung zur Verwendung gemäß Anspruch 4, wobei der pathologische Zustand neuronaler Tod, insbesondere bei transienter oder permanenter Hypoxie-Ischämie, ist.

9. Verbindung zur Verwendung gemäß Anspruch 4, wobei der pathologische Zustand neuronaler Tod, insbesondere bei Verschluss der mittleren Hirnarterie (Middle Cerebral Artery Occlusion (MCAO)), ist.

## Revendications

1. Composé approprié pour l'inhibition de l'activité caspase-2 ayant la SEQ ID N° 5 : ester de quinolinylcarbonyl-L-valinyl-L-aspartyl(ester méthylique)-L-valinyl-L-alaninyl-L-aspartyl(ester méthylique)2,6-difluorophényle.

2. Composition pharmaceutique comprenant une quantité thérapeutiquement efficace du composé selon la revendication 1, en association avec un support pharmaceutiquement acceptable.

3. Composé ayant la SEQ ID N°5 : ester de quinolinylcarbonyl-L-valinyl-L-aspartyl(ester méthylique)-L-valinyl-L-alaninyl-L-aspartyl(ester méthylique)2,6-difluorophényle pour une utilisation en tant que médicament.

4. Composé ayant la SEQ ID N°5 : ester de quinolinylcarbonyl-L-valinyl-L-aspartyl(ester méthylique)-L-valinyl-L-alaninyl-L-aspartyl(ester méthylique)2,6-difluorophényle destiné à une utilisation dans le traitement d'un état pathologique incluant les lésions d'hypoxie-ischémie, H-I, et les états de type accident vasculaire cérébral.

5. Composé destiné à une utilisation selon la revendication 4, dans lequel la lésion d'H-I est une hypoxie-ischémie cérébrale.

6. Composé destiné à une utilisation selon la revendication 4, dans lequel l'état pathologique est la mort neuronale, en particulier dans une hypoxie-ischémie globale ou focale.

7. Composé destiné à une utilisation selon la revendication 4, dans lequel l'état pathologique est la mort neuronale, en particulier dans une hypoxie-ischémie chez l'adulte ou néonatale.

8. Composé destiné à une utilisation selon la revendication 4, dans lequel l'état pathologique est la mort neuronale, en particulier dans une hypoxie-ischémie transitoire ou permanente.

9. Composé destiné à une utilisation selon la revendication 4, dans lequel l'état pathologique est la mort neuronale, en particulier dans une occlusion de l'artère cérébrale moyenne (MCAO).
